(19) Europäisches Patentamt
European Patent Office
Office européen des brevets

(11) EP 3 237 409 B1

(12) **EUROPÄISCHE PATENTSCHRIFT**

(45) Veröffentlichungstag und Bekanntmachung des Hinweises auf die Patenterteilung:
**05.01.2022 Patentblatt 2022/01**

(21) Anmeldenummer: **15801678.2**

(22) Anmeldetag: **24.11.2015**

(51) Int Cl.:
***C07D 405/14*** (2006.01) ***C07D 409/14*** (2006.01)
***H01L 51/00*** (2006.01)

(86) Internationale Anmeldenummer:
**PCT/EP2015/002360**

(87) Internationale Veröffentlichungsnummer:
**WO 2016/102040 (30.06.2016 Gazette 2016/26)**

(54) **CARBAZOLE MIT ZWEI DIBENZOFURAN- ODER DIBENZOTHIOPHENSUBSTITUENTEN**

CARBAZOLES WITH TWO DIBENZOFURAN OR DIBENZOTHIOPHENE SUBSTITUENTS

CARBAZOLES COMPORTANT DEUX SUBSTITUANTS DIBENZOFURANNE OU DIBENZOTHIOPHÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2014 EP 14004391**

(43) Veröffentlichungstag der Anmeldung:
**01.11.2017 Patentblatt 2017/44**

(73) Patentinhaber: **Merck Patent GmbH**
**64293 Darmstadt (DE)**

(72) Erfinder:
- **PARHAM, Amir Hossain**
  **60486 Frankfurt am Main (DE)**
- **EBERLE, Thomas**
  **76829 Landau (DE)**
- **JATSCH, Anja**
  **60489 Frankfurt am Main (DE)**
- **GROSSMANN, Tobias**
  **64297 Darmstadt (DE)**
- **KROEBER, Jonas Valentin**
  **60311 Frankfurt am Main (DE)**

(56) Entgegenhaltungen:
**WO-A1-2008/146838** **WO-A1-2009/060757**
**WO-A1-2011/004639** **JP-A- 2011 008 991**

- **None**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 237 409 B1

**Beschreibung**

Die vorliegende Erfindung betrifft Carbazole mit zwei Dibenzofuran- oder Dibenzothiophensubstituenten, welche sich für den Einsatz in elektronischen Vorrichtungen eignen gemäß Anspruch 1. Weiterhin betrifft die vorliegende Erfindung eine elektronische Vorrichtung gemäß Anspruch 13.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische FeldeffektTransistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:

(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Elektronische Vorrichtungen, die Carbazole mit Dibenzofuran- oder Dibenzothiophen-Substituenten enthalten, sind unter anderem aus den Veröffentlichungen WO2008/146838, JP2011008991, WO2009/060757, WO2011/004639, US 2012/0289708, WO 2012/086170, US 2012/071668, US 2012/091887, WO 2012/033108, CN 102850334, WO 2013/032278, US 2012/0256169, WO 2012/036482; WO 2013/5923, WO 2013/084885, WO 2013/102992, WO 2013/084881, WO 2012/067425, US 2011/260138,WO 2011/125680, WO 2011/122132, WO 2013/109045, WO 2013/151297, WO 2013/41176, WO 2012/108389, KR 2013/0025087 und KR 2013/0112342 bekannt.

Bekannte elektronische Vorrichtungen weisen ein brauchbares Eigenschaftsprofil auf. Allerdings besteht die dauerhafte Notwendigkeit, die Eigenschaften dieser Vorrichtungen zu verbessern.

Zu diesen Eigenschaften gehört insbesondere die Energieeffizienz, mit der eine elektronische Vorrichtung die vorgegebene Aufgabe löst. Bei organischen Leuchtdioden, die sowohl auf niedermolekularen Verbindungen als auch auf polymeren Materialien basieren können, sollte insbesondere die Lichtausbeute hoch sein, so dass zum Erreichen eines bestimmten Lichtflusses möglichst wenig elektrische Leistung aufgebracht werden muss. Weiterhin sollte auch zum Erzielen einer vorgegebenen Leuchtdichte eine möglichst geringe Spannung notwendig sein. Ein weiteres Problem stellt insbesondere die Lebensdauer der elektronischen Vorrichtungen dar.

Aufgabe der vorliegenden Erfindung ist daher die Bereitstellung neuer Verbindungen, welche zu Elektronischen Vorrichtungen mit verbesserten Eigenschaften führen. Insbesondere ist die Aufgabe Lochtransportmaterialien, Lochinjektionsmaterialien, Lochblockiermaterialien, Elektroneninjektionsmaterialien, Elektronenblockiermaterialien und/oder Elektronentransportmaterialien bereitzustellen, welche verbesserte Eigenschaften in Bezug auf Effizienz, Betriebsspannung und/oder Lebensdauer zeigen. Weiterhin sollten sich die Verbindungen möglichst einfach verarbeiten lassen, insbesondere eine gute Löslichkeit und Filmbildung zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Verbindungen mit allen Merkmalen des Patentanspruchs 1 gelöst werden. Zweckmäßige Abwandlungen der erfindungsgemäßen Verbindungen werden in den auf Anspruch 1 rückbezogenen abhängigen Ansprüchen unter Schutz gestellt.

Gegenstand der Erfindung ist somit eine Verbindung, umfassend Strukturen der Formel (I)

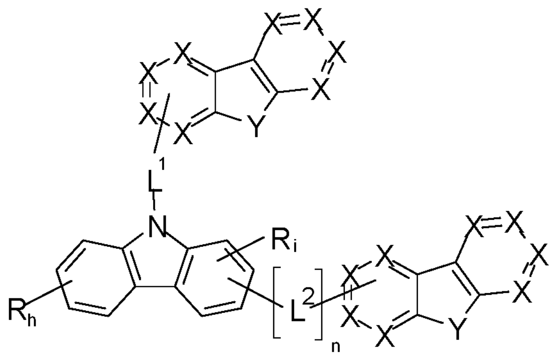

Formel (I)

wobei für die verwendeten Symbole gilt:

X ist $CR^1$ oder C für die Anbindungsstelle der Reste $L^1$, $L^2$ oder der Carbazolgruppe;

Y ist bei jedem Auftreten gleich oder verschieden O oder S;

$L^1$, $L^2$ ist ein aromatisches Ringsystem mit 6 bis 40 C-Atomen, das keine kondensierten aromatischen Ringe (bspw. Naphthaline, Anthracene, Benzanthracene oder Pyrene) aufweist, wobei das aromatische Ringsystem durch einen oder mehrere Reste $R^4$ substituiert sein kann, vorzugsweise ist die Gruppe $L^1$, $L^2$ eine Arylgruppe mit 6 bis 40 C-Atomen, die keine kondensierten aromatischen Ringe aufweist, wobei die Arylgruppe durch einen oder mehrere Reste $R^4$ substituiert sein kann wobei die Substituenten $R^4$ ebenfalls keine kondensierten aromatischen Ringe umfassen und ebenfalls keine heteroaromatischen Ringsysteme oder Heteroarylgruppen umfassen;
einer der Reste R ist eine Carbazolgrupe, die nicht über den Stickstoff der Carbazolgruppe gebunden ist und die jeweils durch einen oder mehrere Reste $R^2$ substituiert sein kann, und alle anderen Reste R sind gleich H;

$R^1$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^2)_3$, $B(OR^2)_2$, $OSO_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C{=}CR^2$, C=C , $Si(R^2)_2$, $Ge(R^2)_2$, $Sn(R^2)_2$, C=O, C=S, C=Se, $P(=O)(R^2)$, SO, $SO_2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;

$R^2$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch C=C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxy-gruppe mit 5 bis 40 aromatischen Ringatomen, die durch einen oder mehrere Reste $R^3$ substituiert sein kann, oder eine Kombination dieser Systeme; dabei können zwei oder mehrere benachbarte Sub-

stituenten $R^2$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können auch zwei Reste $Ar^1$, welche an dasselbe Phosphoratom binden, durch eine Einfachbindung oder eine Brücke, ausgewählt aus $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ und $P(=O)R^3$, miteinander verknüpft sein;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ring-system bilden;

$R^4$ ist bei jedem Auftreten gleich oder verschieden H, D, F, Cl, Br, I, CHO, $C(=O)Ar^2$, $P(=O)(Ar^2)_2$, $S(=O)Ar^2$, $S(=O)_2Ar^2$, CN, $NO_2$, $Si(R^5)_3$, $B(OR^5)_2$, $OSO_2R^5$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch C=C , $Si(R^5)_2$, $Ge(R^5)_2$, $Sn(R^5)_2$, C=O, C=S, C=Se, $P(=O)(R^5)$, SO, $SO_2$, O, S oder $CONR^5$ ersetzt sein können und wobei ein oder mehrere H-Atome durch D, F, Cl, Br, I, CN oder $NO_2$ ersetzt sein können, oder ein aromatisches Ringsystem mit 5 bis 40 C-Atomen, das keine kondensierten aromatischen Ringe aufweist und das jeweils durch einen oder mehrere Reste $R^5$ substituiert sein kann, oder eine Aryloxygruppe mit 5 bis 40 aromatischen Ringatomen, die durch einen oder mehrere Reste $R^5$ substituiert sein kann, oder eine Kombination dieser Systeme, dabei können zwei oder mehrere benachbarte Substituenten $R^4$ auch miteinander ein mono- oder polycyclisches, aliphatisches Ringsystem bilden, das jedoch keine kondensierten aromatischen Ringe aufweist;

$Ar^2$ ist bei jedem Auftreten gleich oder verschieden ein aromatisches Ringsystem mit 5 bis 30 C-Atomen, das keine kondensierten aromatischen Ringe aufweist und das mit einem oder mehreren Resten $R^5$ substituiert sein kann;

$R^5$ ist bei jedem Auftreten gleich oder verschieden H, D, F oder ein aliphatischer und/oder aromatischer Kohlenwasserstoffrest mit 1 bis 20 C-Atomen, in dem auch H-Atome durch F ersetzt sein können; dabei können zwei oder mehrere benachbarte Substituenten $R^5$ auch miteinander ein mono- oder polycyclisches, aliphatisches Ringsystem bilden, das jedoch keine kondensierten aromatischen Ringe aufweist;

h ist 1;

i ist 0;

n ist 0 oder 1.

mit der Maßgabe, dass
die Gruppe $L^1$ und/oder $L^2$ mit dem Carbazolring der Formel (I) und den zwischen der Dibenzofuranstruktur (Y=O) und/oder der Dibenzothiophenstruktur (Y=S) eine durchgängige Konjugation ausbildet.

Die Gruppe $L^1$ und/oder $L^2$ bildet mit dem Carbazolring der Formel (I) und der Dibenzofuranstruktur (Y=O) und/oder der Dibenzothiophenstruktur (Y=S) eine durchgängige Konjugation aus. Eine durchgängige Konjugation der aromatischen beziehungsweise heteroaromatischen Systeme wird ausgebildet sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen dem Carbazolring der Formel (I) und der Dibenzofuranstruktur (Y=O) und/oder der Dibenzothiophenstruktur (Y=S) liegt. Im Gegensatz hierzu kann bei einer Spirobifluorenstruktur eine durchgängige Konjugation ausgebildet werden, falls die Verbindung zwischen dem Carbazolring der Formel (I) und der Dibenzofuranstruktur (Y=O) und/oder der Dibenzothiophenstruktur (Y=S) über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die die Verbindung zwischen dem Carbazolring der Formel (I) und der Dibenzofuranstruktur (Y=O) und/oder der Dibenzothiophenstruktur

(Y=S) über verschiedene Phenylgruppen der Spirobifluorenstruktur erfolgt, die über das $sp^3$ hybridisierte Kohlenstoffatom in Position 9 verbunden sind, ist die Konjugation unterbrochen.

Dabei bedeutet "benachbarte Kohlenstoffatome", dass die Kohlenstoffatome direkt aneinander gebunden sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Die folgenden Definitionen sollen gelten, sofern keine andere Definition oder Einschränkung angegeben werden.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden. Ferner stellen auch Systeme, in denen zwei oder mehrere aromatische oder heteroaromatische Ringe direkt aneinander gebunden sind, wie z. B. Biphenyl oder Terphenyl, Aryl- oder Heteroarylgruppen dar.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$-bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$-bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Es hat sich gezeigt, dass Verbindungen der Formel (I), die in elektronischen Vorrichtungen verwendet werden, besonders vorteilhaft insbesondere hinsichtlich der Lebensdauer der Vorrichtung sind.

Die verbindende Gruppe $L^1$ oder $L^2$ in Formel (I) weist keine kondensierten aromatischen Ringe auf, so dass beispielsweise keine Naphthlyen-Gruppen enthalten sind. Dies schließt Substituenten an den Gruppen $L^1$ oder $L^2$ ein, so dass die Substituenten $R^4$ ebenfalls keine kondensierten aromatischen Ringe, wie Naphthly-Gruppen, umfassen.

Darüber hinaus umfasst die verbindende Gruppe $L^1$ oder $L^2$ in Formel (I) keine heteroaromatischen Ringsysteme oder Heteroarylgruppen, beispielsweise Carbazolgruppen oder Pyridingruppen. Dies schließt Substituenten an den Gruppen $L^1$ oder $L^2$ ein, so dass die Substituenten $R^4$ ebenfalls keine heteroaromatischen Ringsysteme oder Heteroarylgruppen, wie Carbazolgruppen oder Pyridingruppen, umfassen.

Besonders bevorzugt umfasst mindestens einer dieser Gruppen $L^1$ oder $L^2$ mindestens eine Phenylen- und/oder Biphenylen-Gruppe, die mit einem oder mehreren Resten $R^4$ substituiert sein können. Besonders vorteilhaft für die Leistungsdaten elektronischer Vorrichtungen ist, wenn $L^1$ eine Phenylengruppe ist, die mit einem oder mehreren Resten $R^4$ substituiert sein kann. Weiterhin bevorzugt ist, wenn $L^1$ und $L^2$ eine Phenylengruppe darstellen, die jeweils mit einem oder mehreren Resten $R^4$ substituiert sein können.

Es hat sich weiterhin als vorteilhaft gezeigt, wenn die Dibenzofuran- oder Dibenzothiophengruppe, die über die Gruppe $L^1$ an das Stickstoffatom in Position 9 des Carbazols in Formel (I) gebunden ist, keine weiteren Carbazole, Dibenzofurane oder Dibenzothiophene enthält.

Weiterhin vorteilhaft ist es, wenn sowohl die Dibenzofuran- oder Dibenzothiophengruppe, die über die Gruppe $L^1$ an das Stickstoffatom in Position 9 des Carbazols in Formel (I) gebunden ist, als auch die Dibenzofuran- oder Dibenzothiophengruppe, die über die Gruppe $L^2$ an eine der Positionen 1 bis 4 des Carbazols in Formel (I) gebunden ist, keine weiteren Carbazole, Dibenzofurane oder Dibenzothiophene enthalten.

Eine Arylgruppe umfasst zwei oder mehr kondensierte Ringe, falls mindestens zwei Ringe jeweils zwei Ringatome gemeinsam haben und diese Ringe jeweils aromatisch sind. Vorzugsweise treten die aromatischen Kerne von mindestens zwei Ringen in größerem Maße in Wechselwirkung, wobei diese Wechselwirkung über spektroskopische Methodern, unter anderem Änderungen der Fluoreszenz, Phosphoreszenz oder einer Verschiebung im UV-Spektrum nachweisbar sind. So umfasst beispielsweise Fluoren lediglich zwei nicht kondensierte Ringe, während Dibenzofuran oder Dibenzothiofuran heteroaromatische Systeme mit drei kondensierten Ringen darstellen. Anthracen, Fluoranthen und andere aromatische Systeme, die drei oder mehr aromatische Ringe mit jeweils 2 gemeinsamen Kohlenstoffatomen pro Ring aufweisen, stellen kondensierte Systeme dar, die mindestens 3 Ringe aufweisen. Vereinfacht kann dargelegt werden, dass kondensierte aromatische Systeme, im Wesentlichen aus $sp^2$-hybridisierten Ringkohlenstoffatomen aufgebaut sind. Eine vergleichbare Definition gilt für Heteroarylgruppen.

In einer bevorzugten Ausgestaltung kann vorgesehen sein, dass die Summe von sämtlichen Indices h und i gleich 1 ist.

Es kann mindestens eine der Gruppen $L^1$ oder $L^2$ in Formel (I) mindestens eine Phenylen-, oder Biphenyl-Gruppe umfassen, wobei eine Phenylengruppe besonders bevorzugt ist.

Darüber hinaus sind Verbindungen bevorzugt, die dadurch gekennzeichnet sind, dass die Gruppen $L^1$ oder $L^2$ in Formel (I) insgesamt höchstens 36, vorzugsweise höchstens 24, besonders bevorzugt höchstens 12 und ganz bevorzugt höchstens 6 Kohlenstoffatome aufweisen.

Bevorzugte Verbindungen mit Strukturen gemäß Formel (I), bzw. die zuvor und nachfolgend aufgeführten bevorzugten Ausführungsformen zeichnen sich vorzugsweise dadurch aus, dass mindestens ein Y in Struktur gemäß Formel (I) für O steht, vorzugsweise beide Y in Struktur gemäß Formel (I) für O stehen.

Ferner kann vorgesehen sein, dass mindestens ein Y in Struktur gemäß Formel (I) für S steht.

Bevorzugt sind Verbindungen umfassend Strukturen der Formel (I), in denen mindestens ein Rest $R^1$ für eine Gruppe steht, die ausgewählt ist aus den Formeln ($R^1$-1) bis ($R^1$-22)

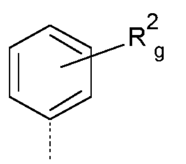

Formel ($R^1$-1)

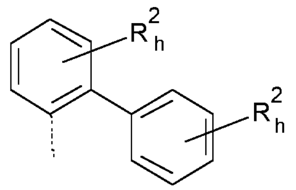

Formel ($R^1$-2)

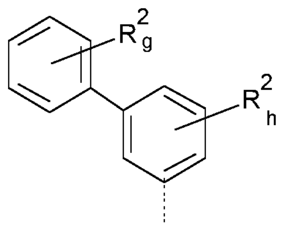

Formel ($R^1$-3)

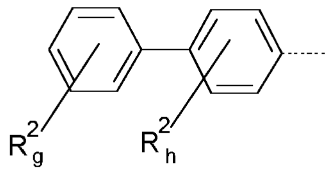

Formel ($R^1$-4)

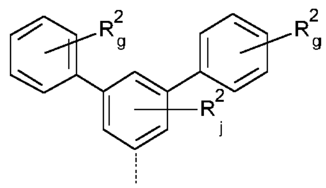

Formel ($R^1$-5)

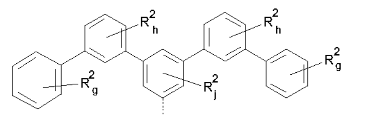

Formel ($R^1$-6)

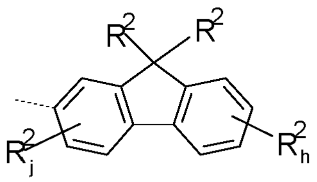

Formel ($R^1$-7)

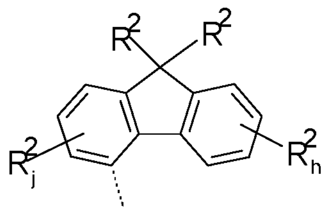

Formel ($R^1$-8)

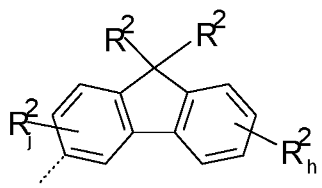

Formel ($R^1$-9)

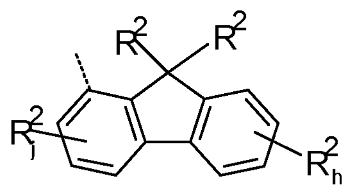

Formel ($R^1$-10)

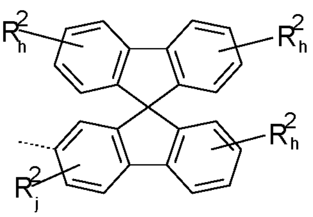

Formel ($R^1$-11)

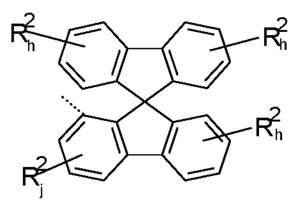

Formel ($R^1$-12)

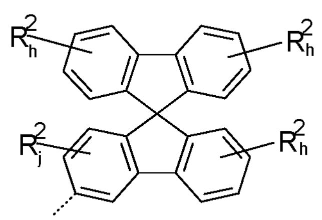

Formel ($R^1$-13)

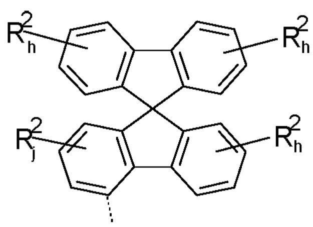

Formel ($R^1$-14)

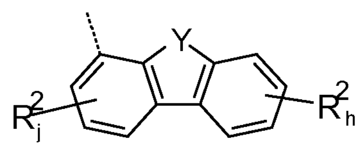

Formel ($R^1$-15)

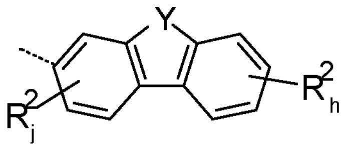

Formel ($R^1$-16)

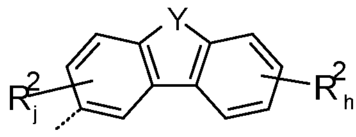

Formel ($R^1$-17)

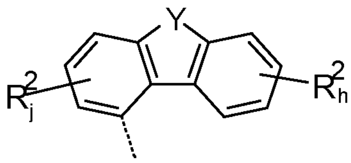

Formel ($R^1$-18)

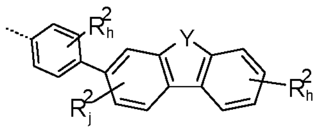

Formel ($R^1$-19)

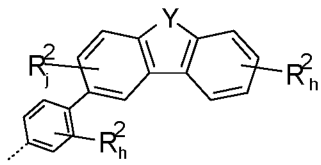

Formel ($R^1$-20)

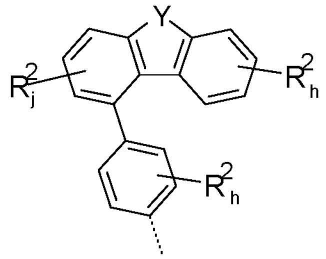

Formel ($R^1$-21)

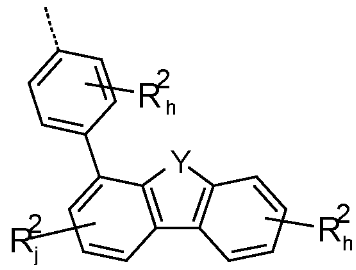

Formel ($R^1$-22)

, wobei die gestrichelte Bindung die Anbindungsposition markiert, g 0, 1, 2, 3, 4 oder 5 ist, h 0, 1, 2, 3 oder 4 ist, j 0, 1, 2 oder 3 ist, $R^2$ und Y die zuvor genannte Bedeutung aufweisen.

Es ist bevorzugt, wenn der Rest $R^1$ keinen Carbazolring darstellt.

Vorzugsweise kann vorgesehen sein, dass die Summe der Indices g, h und j in den Strukturen der Formel ($R^1$-1) bis ($R^1$-22) jeweils höchstens 3, vorzugsweise höchstens 2 und besonders bevorzugt höchstens 1 beträgt.

Bevorzugt sind Verbindungen umfassend Strukturen der Formel (I), wobei in Struktur gemäß Formel (I) mindestens ein Rest $R^1$ für eine Gruppe steht, die ausgewählt ist aus den Formeln ($R^1$-23) bis ($R^1$-25)

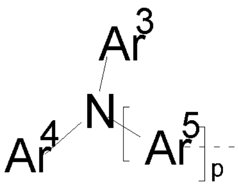

Formel ($R^1$-23)

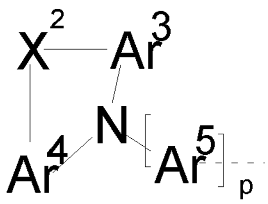

Formel ($R^1$-24)

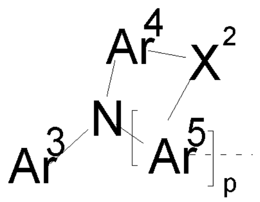

Formel ($R^1$-25)

, wobei die gestrichelte Bindung die Anbindungsposition markiert und

$Ar^3$, $Ar^4$, $Ar^5$ jeweils unabhängig ein aromatisches Ringsystem, vorzugsweise eine Arylgruppe, mit 6 bis 40 C-Atomen oder ein heteroaromatisches Ringsystem, vorzugsweise eine Heteroarylgruppe, mit 3 bis 40 C-Atomen, welches jeweils durch einen oder mehrere Reste $R^1$ substituiert sein kann;

p 0 oder 1 ist und

$X^2$ für eine Bindung, $CR^1_2$, C=O, $N(R^1)$, $B(R^1)$, $SiR^1_2$, O oder S, vorzugsweise $CR^1_2$, C=O, $N(Ar^1)$, O oder S steht, wobei die Reste $R^1$ und $Ar^1$ die zuvor genannten Bedeutungen aufweisen.

Bevorzugt sind Verbindungen umfassend Strukturen der Formeln (I), wobei in Struktur gemäß Formel (I) mindestens ein Gruppe ausgewählt aus $L^1$, $L^2$ für eine Gruppe steht, die der Formel (L-1) entspricht. Alternative Gruppen $L^1$ und $L^2$ sind ausgewählt aus den Formeln (L-1) bis (L-12),

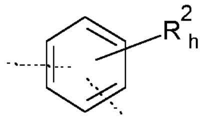

Formel (L-1)

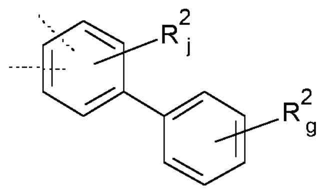

Formel (L-2)

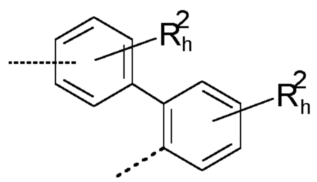

Formel (L-3)

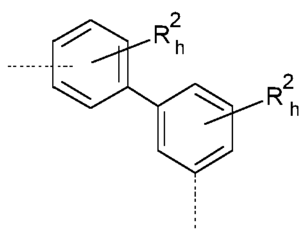

Formel (L-4)

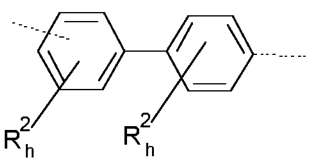

Formel (L-5)

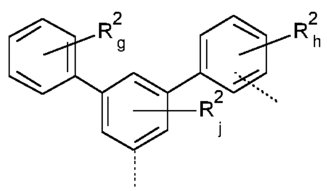

Formel (L-6)

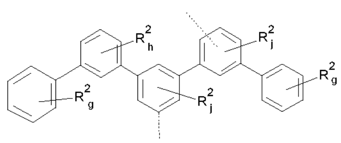

Formel (L-7)

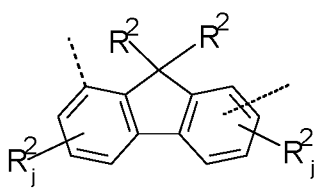

Formel (L-8)

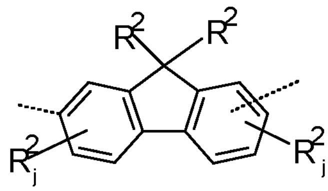

Formel (L-9)

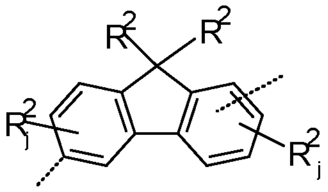

Formel (L-10)

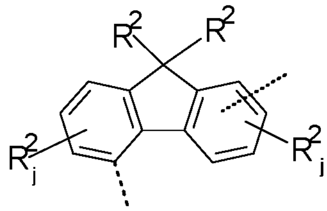

Formel (L-11)

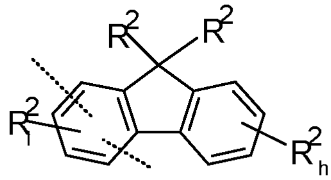

Formel (L-12)

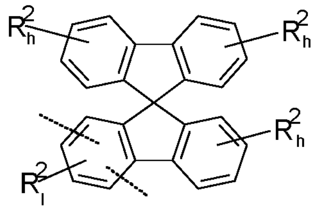

Formel (L-13)

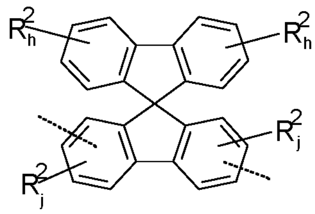

Formel (L-14)

, wobei die gestrichelten Bindungen jeweils die Anbindungspositionen markieren, der Index l 0, 1 oder 2 ist, der Index g 0, 1, 2, 3, 4 oder 5, vorzugsweise 0, 1, 2, oder 3, besonders bevorzugt 0, 1 oder 2 ist, der Index h 0, 1, 2, 3 oder 4, vorzugsweise 0, 1, oder 2, besonders bevorzugt 0 oder 1 ist, der Index j 0, 1, 2 oder 3, vorzugsweise 0, 1, oder 2, besonders bevorzugt 0 oder 1 ist, und $R^2$ die zuvor genannten Bedeutungen aufweist.

Vorzugsweise ist die Summe der Indices h, j, g und l in einer Struktur gemäß den Formeln (L-1) bis (L-14) kleiner oder gleich 5, vorzugsweise 0, 1, 2 oder 3 und besonders bevorzugt 0 oder 1.

Besonders bevorzugte Verbindungen umfassen Strukuren gemäß den folgenden Formeln (II), (III), (IV), (V),

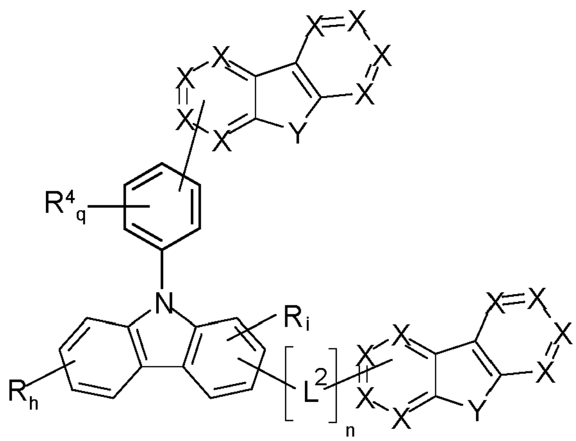

Formel (II)

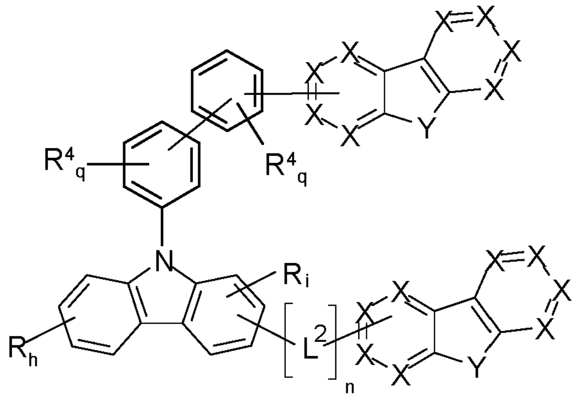

Formel (III)

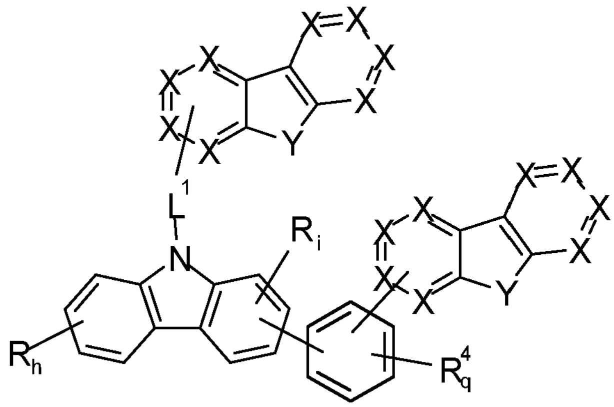

Formel (IV)

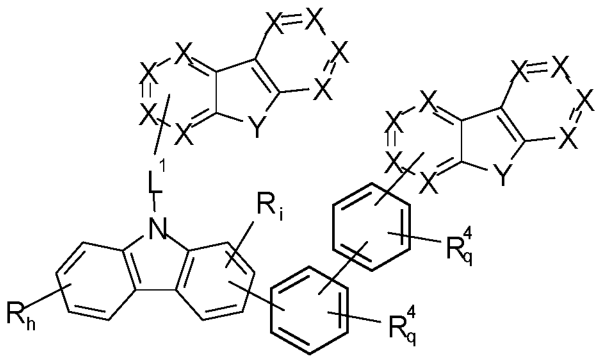

Formel (V)

, wobei die dargelegten Symbole X, Y, R, $R^4$, $L^1$, $L^2$ und die Indices h, i und n die in zuvor dargelegten Bedeutungen haben und der Index q 0, 1, 2, 3 oder 4, vorzugsweise 0, 1, 2 oder 3 und besonders bevorzugt 0, 1, oder 2 bedeutet.

Ferner kann vorgesehen sein, dass die Reste $R^1$, $R^2$ und $R^3$ in Summe höchstens 4, vorzugsweise höchstens 3, besonders bevorzugt höchstens 2, speziell bevorzugt höchstens 1 und ganz besonders bevorzugt kein Stickstoffatom aufweisen.

Besonders bevorzugt sind Verbindungen mit Strukturen gemäß Formel (II) bei denen die Gruppe $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind, und wobei die Struktur der Formel (L-1) ganz besonders bevorzugt ist. Ferner sind Verbindungen mit Strukturen gemäß Formel (II) bevorzugt bei denen n = 0 ist, so dass eine Bindung zwischen dem Carbazolring und der Dibenzofuran- oder Dibenzothiophen-Struktur vorgesehen ist.

Besonders bevorzugt sind Verbindungen mit Strukturen gemäß Formel (III) bei denen die Gruppe $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist. Ferner sind Verbindungen mit Strukturen gemäß Formel (III) bevorzugt bei denen n = 0 ist, so dass eine Bindung zwischen dem Carbazolring und der Dibenzofuran- oder Dibenzothiophen-Struktur vorgesehen ist.

Besonders bevorzugt sind Verbindungen mit Strukturen gemäß Formel (IV) bei denen die Gruppe $L^1$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonder bevorzugt ist.

Besonders bevorzugt sind Verbindungen mit Strukturen gemäß Formel (V) bei denen die Gruppe $L^1$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugte Verbindungen umfassen Strukuren gemäß den folgenden Formeln (VI), (VII), (VIII), (IX), (X), (XI), (XII) und/oder (XIII)

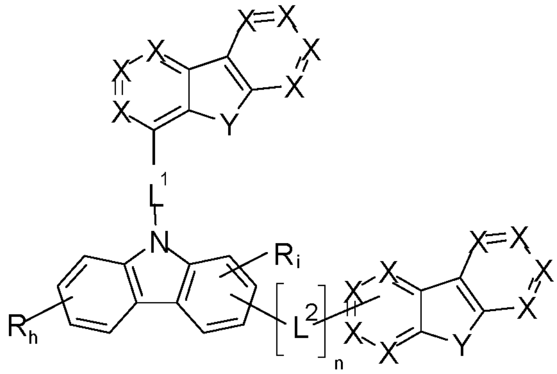

Formel (VI)

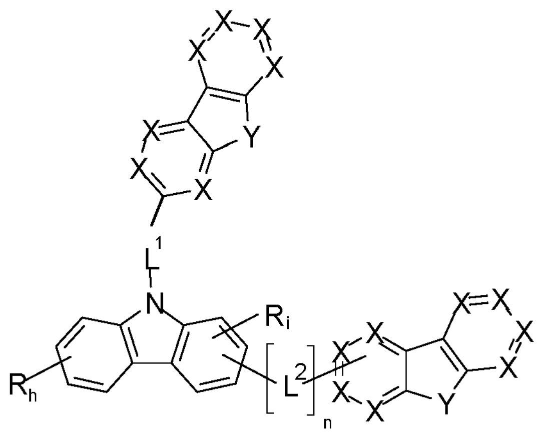

Formel (VII)

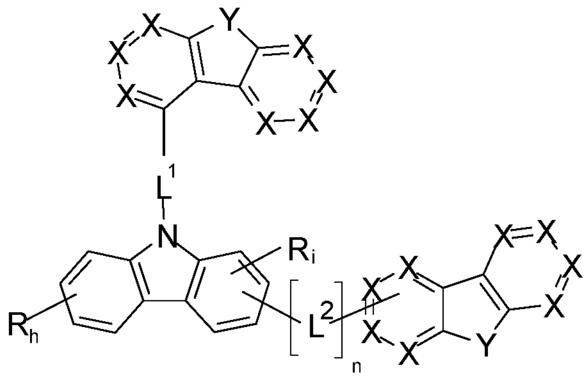

Formel (VIII)

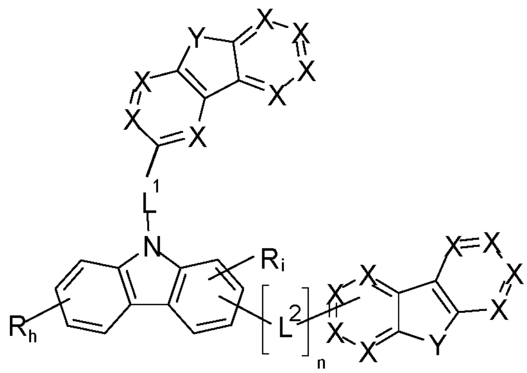

Formel (IX)

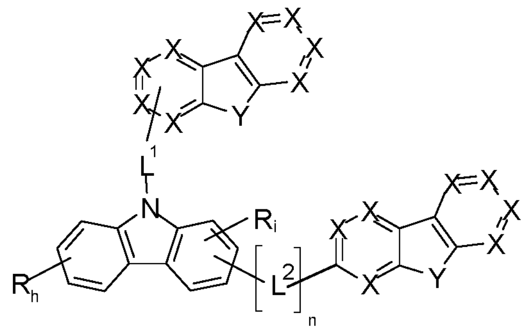

Formel (X)

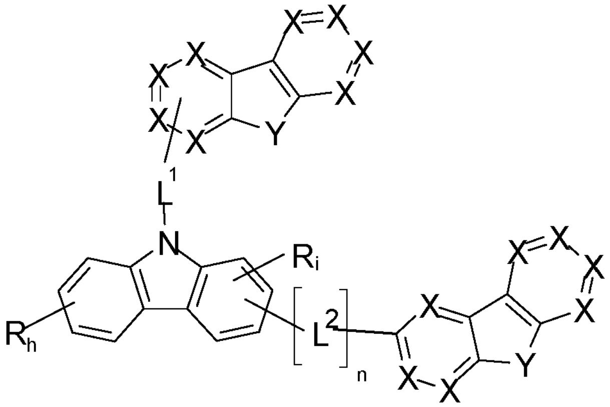

Formel (XI)

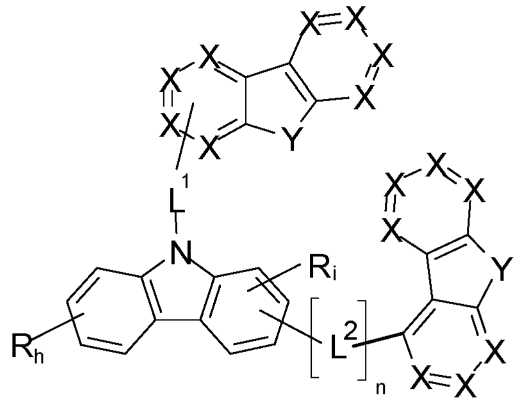

Formel (XII)

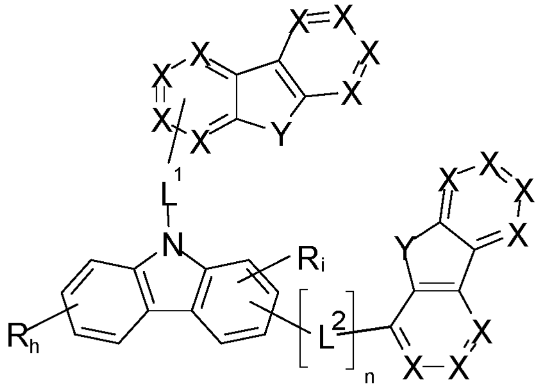

Formel (XIII)

, wobei die dargelegten Symbole X, Y, R, $L^1$, $L^2$ und die Indices h, i und n die in zuvor dargelegten Bedeutungen haben.

Besonders bevorzugt sind Verbindungen mit Strukturen gemäß Formel (VI) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (VII) bei denen mindestens eine der Gruppen $L^1$ und/oder $L^2$ eine Struktur gemäß Formel Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (VIII) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (VIII) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (IX) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (X) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (XI) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und

Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (XI) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (XII) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Besonders bevorzugt sind Verbindungen mit Strukturen gemäß Formel (XIII) bei denen mindestens eine der Gruppen $L^1$ und/oder $L^2$ eine Struktur gemäß Formel (L-1), (L-2), (L-3), (L-4), (L-5), (L-6) und/oder (L-7) aufweist und Strukturen gemäß Formel (L-1), (L-2), (L-3), (L-4) und/oder (L-5) besonders bevorzugt sind und wobei die Struktur der Formel (L-1) ganz besonders bevorzugt ist.

Vorzugsweise kann eine Verbindung mit Strukturen gemäß Formel (I) sowie bevorzugte Varianten dieser Verbindung gemäß Formeln (II) bis (XIII) Reste $R^1$ umfassen, bei denen diese Reste $R^1$ bei jedem Auftreten gleich oder verschieden bevorzugt ausgewählt sind aus der Gruppe bestehend aus H, D, F, Br, I, CN, $Si(R^2)_3$, $B(OR^2)_2$, $C(=O)Ar^1$, einer geradkettigen Alkylgruppe mit 1 bis 10 C-Atomen oder einer geradkettigen Alkoxygruppe mit 1 bis 10 C-Atomen oder einer Alkenylgruppe mit 2 bis 10 C-Atomen oder einer verzweigten oder cyclischen Alkoxygruppe mit 3 bis 10 C-Atomen oder einer verzweigten oder cyclischen Alkylgruppe mit 3 bis 10 C-Atomen, die jeweils mit einem oder mehreren Resten $R^2$ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste $R^2$ substituiert sein kann; dabei können zwei benachbarte Reste $R^1$ oder $R^1$ mit $R^2$ auch miteinander ein mono- oder polycyclisches, aliphatisches oder aromatisches Ringsystem bilden. Besonders bevorzugt sind diese Reste $R^1$ bei jedem Auftreten gleich oder verschieden ausgewählt aus der Gruppe bestehend aus H, D, F, einer geradkettigen Alkoxygruppe mit 1 bis 6 C-Atomen oder einer verzweigten oder cyclischen Alkoxygruppe mit 3 bis 10 C-Atomen, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen Ringsystem mit 5 bis 24 aromatischen Ringatomen, das jeweils durch einen oder mehrere Reste $R^2$ substituiert sein kann; dabei können zwei benachbarte Reste $R^1$ bzw. $R^1$ mit $R^2$ auch miteinander ein mono- oder polycyclisches, aliphatisches oder aromatisches Ringsystem bilden. Besonders bevorzugt kann mindestens einer der Reste $R^1$ in Formel (I) eine Arylgruppe oder eine Heteroarylgruppe mit 6 bis 18 Kohlenstoffatomen darstellen, die mit bis zu drei Resten $R^2$ substituiert sein kann.

Vorzugsweise kann die Verbindung mit Strukturen gemäß Formel (I) sowie bevorzugte Varianten dieser Verbindung gemäß Formeln (II) bis (XIII) Reste $R^2$ umfassen, bei denen diese Reste $R^2$ bei jedem Auftreten gleich oder verschieden bevorzugt ausgewählt sind aus der Gruppe bestehend aus H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten $R^3$ substituiert sein kann, wobei eine oder mehrere nicht benachbarte $CH_2$-Gruppen durch C=C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können,
oder ein aromatisches oder heteroaromatisches Ringsystem mit 5 bis 24 aromatischen Ringatomen, das jeweils durch einen oder mehrere Reste $R^3$ substituiert sein kann, oder eine Aryloxy- oder Heteroaryloxygruppe mit 5 bis 24 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden. Besonders bevorzugt kann mindestens einer der Reste $R^2$ in Formel (I) oder deren bevorzugten Ausgestaltungen eine Arylgruppe oder eine Heteroarylgruppe mit 6 bis 18 Kohlenstoffatomen darstellen, die mit bis zu drei Resten $R^3$ substituiert sein kann.

Vorzugsweise kann die Verbindung mit Strukturen gemäß Formeln (I) bis (XIII) Reste $R^4$ umfassen, bei denen diese Reste $R^4$ bei jedem Auftreten gleich oder verschieden bevorzugt ausgewählt sind aus der Gruppe bestehend aus H, D, F, Cl, Br, I, CHO, $C(=O)Ar^2$, $P(=O)(Ar^2)_2$, $S(=O)Ar^2$, $S(=O)_2Ar^2$, CN, $NO_2$, $Si(R^5)_3$, $B(OR^5)_2$, $OSO_2R^5$, eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten $R^3$ substituiert sein kann, wobei eine oder mehrere nicht benachbarte $CH_2$-Gruppen durch C=C , $Si(R^5)_2$, $Ge(R^5)_2$, $Sn(R^5)_2$, C=O, C=S, C=Se, $P(=O)(R^5)$, SO, $SO_2$, O, S oder $CONR^5$ ersetzt sein können und wobei ein oder mehrere H-Atome durch D, F, Cl, Br, I, CN oder $NO_2$ ersetzt sein können,
oder ein aromatisches Ringsystem mit 6 bis 24 C-Atomen, das keine kondensierten aromatischen Ringe aufweist und das jeweils durch einen oder mehrere Reste $R^5$ substituiert sein kann, oder eine Aryloxygruppe mit 5 bis 24 C-Atomen,

die durch einen oder mehrere Reste $R^5$ substituiert sein kann, oder eine Kombination dieser Systeme; dabei können zwei oder mehrere benachbarte Substituenten $R^4$ auch miteinander ein mono- oder polycyclisches, aliphatisches Ringsystem bilden, das jedoch keine kondensierten aromatischen Ringe aufweist. Besonders bevorzugt kann mindestens einer der Reste $R^4$ in Formeln (II) bis (XIII) eine Arylgruppe mit 6 bis 18 Kohlenstoffatomen darstellen, die mit bis zu drei Resten $R^5$ substituiert sein kann.

Insbesondere bevorzugt sind solche Verbindungen der Formeln (I) bis (XIII) für die $L^1$ eine Phenylengruppe darstellt, insbesondere eine Phenylengruppe der Formel (L-1).

Besonders bevorzugte Verbindungen umfassen Strukuren gemäß den folgenden Formeln 25 bis 30, 37, 51, 75, 79, 81 bis 89, 124 und 140:

| | | |
|---|---|---|
| 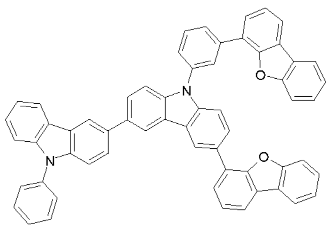 | 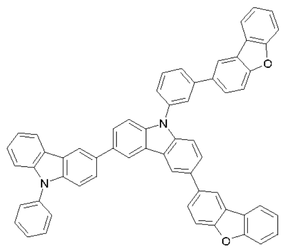 | 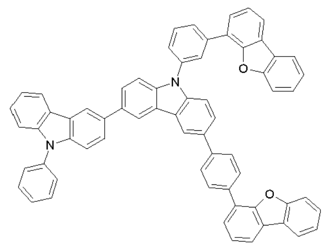 |
| Formel 25 | Formel 26 | Formel 27 |
| 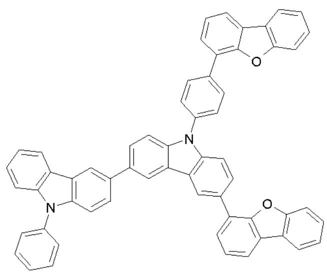 | 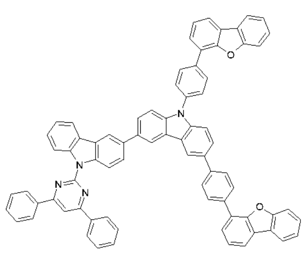 | 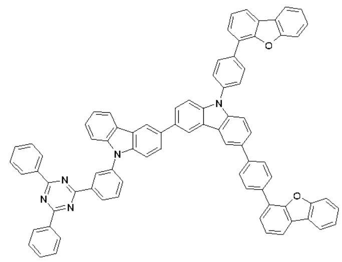 |
| Formel 28 | Formel 29 | Formel 30 |

| | | |
|---|---|---|
| 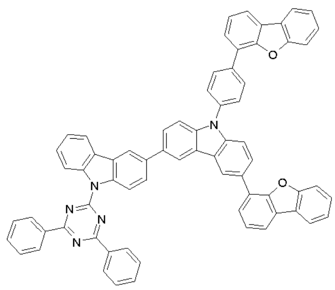 | 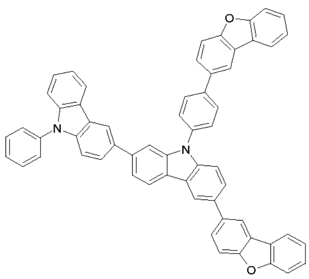 | 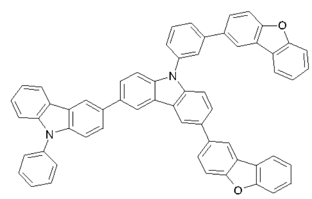 |
| Formel 37 | Formel 51 | Formel 75 |
| 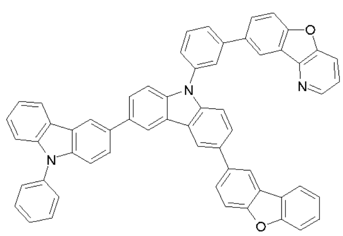 | | 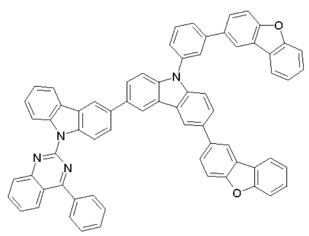 |
| Formel 79 | | Formel 81 |

(fortgesetzt)

| | | |
|---|---|---|
| 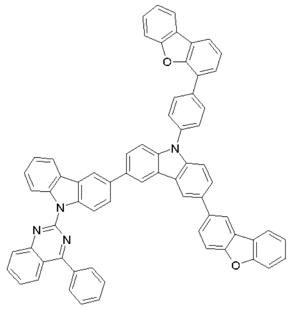 | 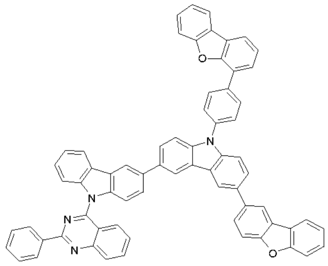 | 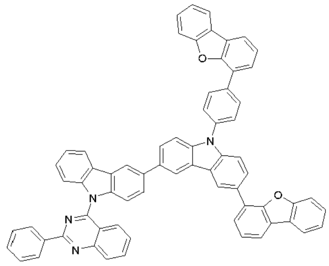 |
| Formel 82 | Formel 83 | Formel 84 |
| 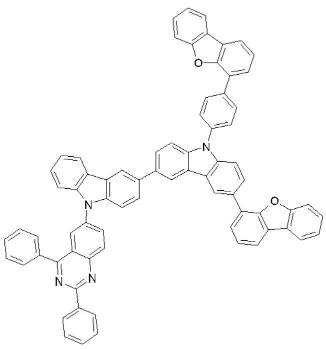 | 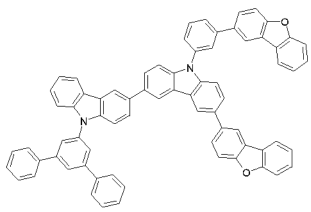 | 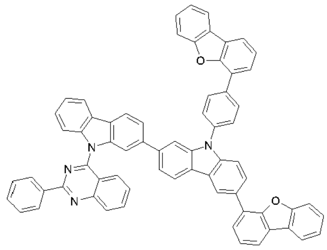 |
| Formel 85 | Formel 86 | Formel 87 |

| | | |
|---|---|---|
| 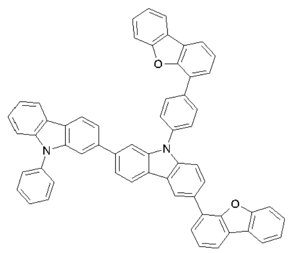 | 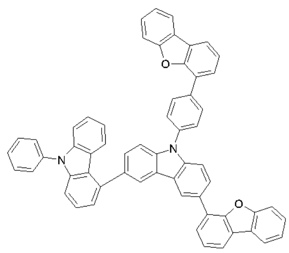 | 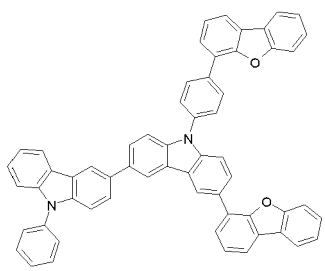 |
| Formel 88 | Formel 89 | Formel 124 |
| 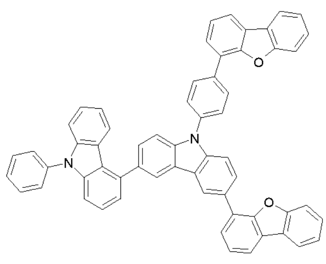 | | |
| Formel 140 | | |

Bevorzugte Ausführungsformen von alternativen Verbindungen werden in den Beispielen näher ausgeführt.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Ein nicht erfindungsgemässer Gegenstand der vorliegenden Beschreibung ist ein Verfahren zur Herstellung von Verbindungen, umfassend Strukturen gemäß Formel (I), das dadurch gekennzeichnet ist, dass in einer Kupplungsreaktion eine Gruppe, umfassend mindestens einen CarbazolRest, mit einer Gruppe, umfassend mindestens einen Benzofuran- und/oder einen Benzothiophen-Rest, verbunden wird.

Besonders geeignete und bevorzugte Kupplungsreaktionen, die alle zu C-C-Verknüpfungen und/oder C-N-Verknüpfungen führen, sind solche gemäß BUCHWALD, ULLMANN, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

In folgendem Synthese-Schema sind die Verbindungen zur Vereinfachung der Strukturen mit einer geringen Anzahl an Substituenten gezeigt. Dies schließt das Vorhandensein von beliebigen weiteren Substituenten in den Verfahren nicht aus.

Eine Umsetzung ergibt sich beispielhaft gemäß folgendem Schema, ohne dass hierdurch eine Beschränkung erfolgen soll. Die Teilschritte des Schemas können hierbei beliebig kombiniert werden.

Beispielsweise kann gemäß folgendem Schema ausgehend von einer reaktiven Carbazol-Verbindung beispielsweise durch eine Suzuki-Umsetzung mit einer reaktiven Dibenzofuran- oder Dibenzothiophen-Verbindung eine Carbazol-Verbindung mit einem Dibenzofuran- oder Dibenzothiophen-Substituenten erhalten werden, die in einem weiteren Schritt, beispielsweise einer Buchwald und/oder Ullmann-Reaktion, zu einer Verbindung gemäß der vorliegenden Erfindung umgesetzt wird.

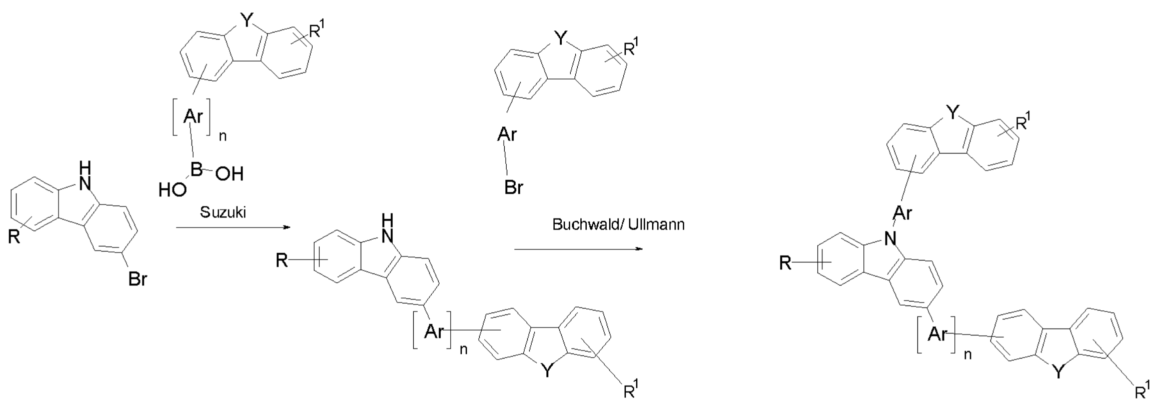


Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) bzw. die zuvor und nachfolgend aufgeführten bevorzugten Ausführungsformen in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Beschreibung sind daher Oligomere, Polymere oder Dendrimere enthaltend eine oder mehrere der oben aufgeführten Strukturen der Formel (I) oder erfindungsgemäße Verbindungen, wobei ein oder mehrere Bindungen der erfindungsgemäßen Verbindungen oder der Strukturen der Formel (I) zum Polymer, Oligomer oder Dendrimer vorhanden sind. Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß gemäß Formel (I) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Ferner können die vorliegenden Verbindungen ein relativ geringes Molekulargewicht aufweisen. Ein weiterer Gegenstand der vorliegenden Beschreibung ist demgemäß eine Verbindung, umfassend Strukturen gemäß Formel (I), die ein Molekulargewicht von vorzugsweise höchstens 10000 g/mol, besonders bevorzugt höchstens 5000 g/mol, insbesondere bevorzugt höchstens 3000 g/mol, speziell bevorzugt höchstens 2000 g/mol und ganz besonders bevorzugt höchstens 1000 g/mol aufweist.

Weiterhin zeichnen sich bevorzugte Verbindungen dadurch aus, dass diese sublimierbar sind. Diese Verbindungen weisen im Allgemeinen eine Molmasse von weniger als ca. 1200 g/mol auf.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005.

Nochmals ein weiterer Gegenstand der vorliegenden Erfindung ist eine Formulierung, enthaltend eine erfindungsgemäße Verbindung und mindestens ein Lösemittel. Eine weitere Verbindung in der Formulierung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Nochmals ein weiterer Gegenstand der vorliegenden Erfindung ist eine Zusammensetzung enthaltend eine erfindungsgemäße Verbindung und wenigstens ein weiteres organisch funktionelles Material, ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

Die vorliegenden Erfindung betrifft daher auch eine Zusammensetzung enthaltend wenigstens eine Verbindung umfassend Strukuren gemäß Formel (I) bzw. die zuvor und nachfolgend aufgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden Beschreibung stellt eine Zusammensetzung dar, enthaltend wenigstens eine Verbindung, umfassend mindestens eine Struktur gemäß Formel (I) sowie wenigstens ein Wide-Band-Gap-Material, wobei unter Wide-Band-Gap-Material ein Material im Sinne der Offenbarung von US 7,294,849 verstanden

wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (I) sowie wenigstens einen phosphoreszierende Emitter, wobei unter dem Begriff phosphoreszierende Emitter auch phosphoreszierende Dotanden verstanden werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem

Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

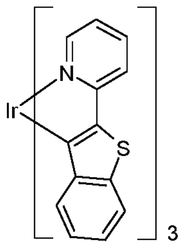
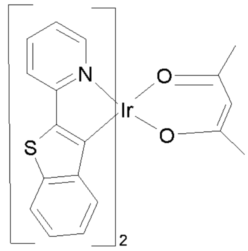
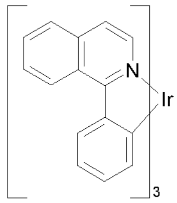
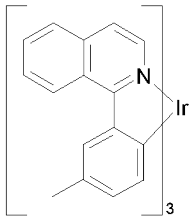
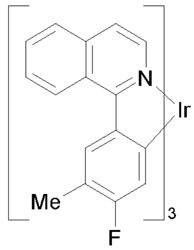
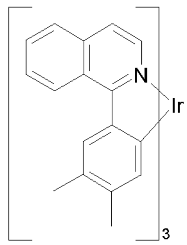
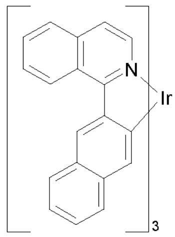
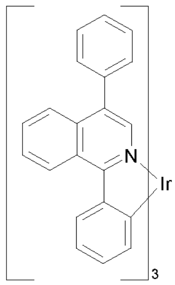
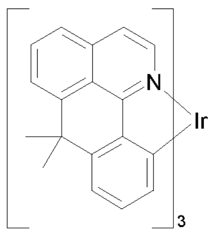
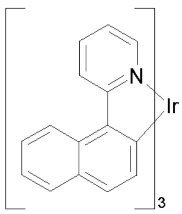
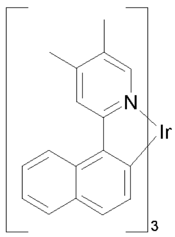
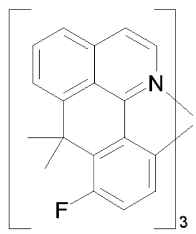
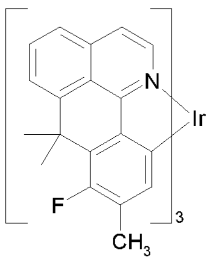
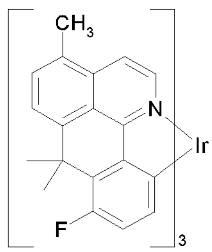
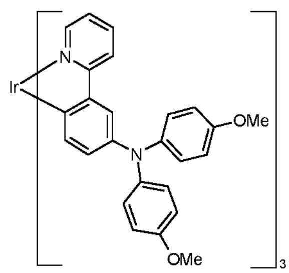

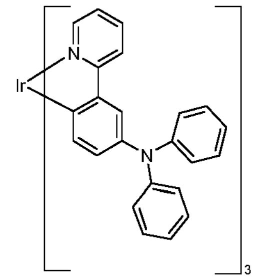
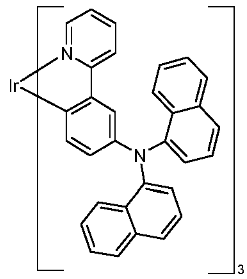
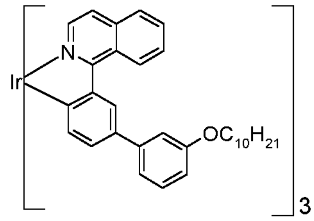
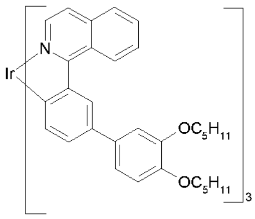
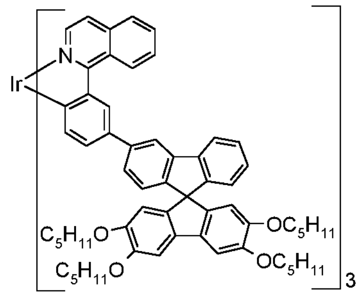
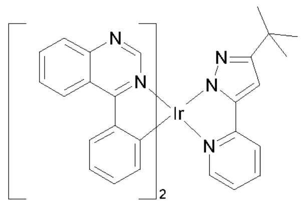
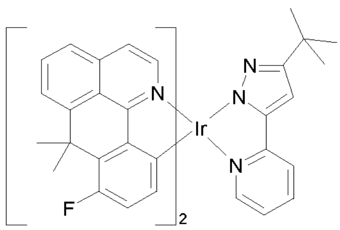
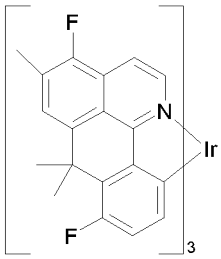
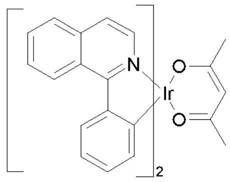
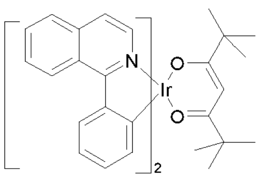
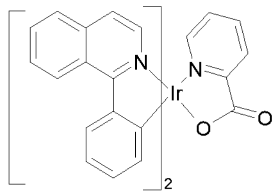
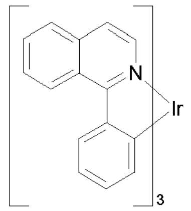
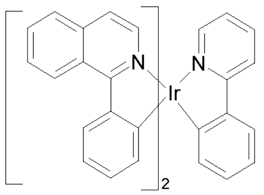
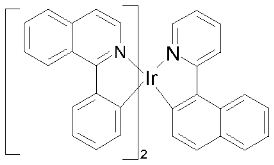
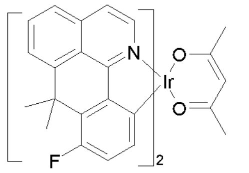
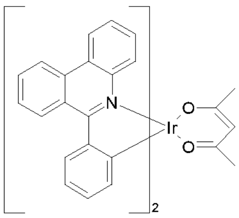
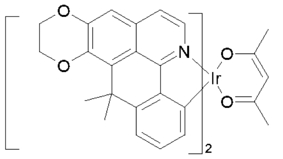
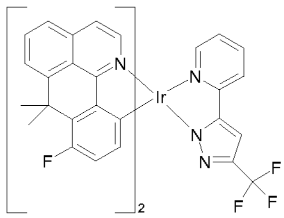

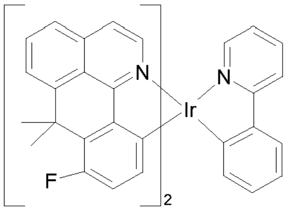
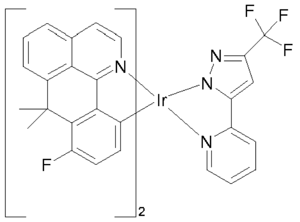
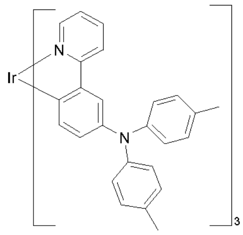

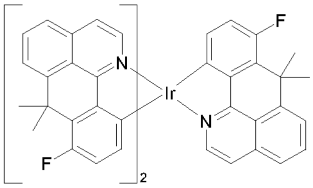
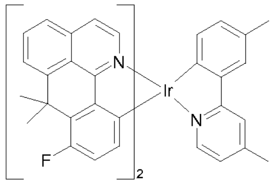
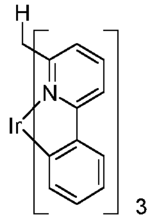

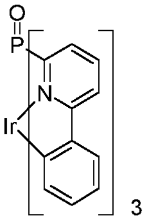
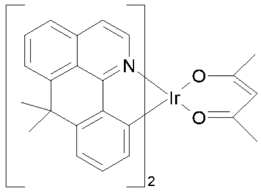
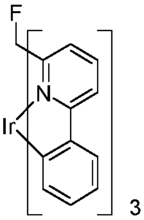

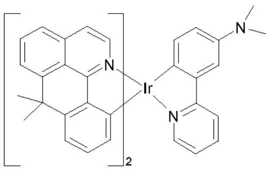
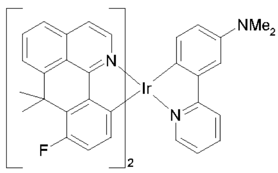
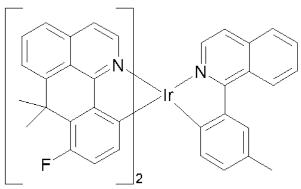

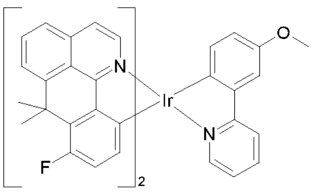
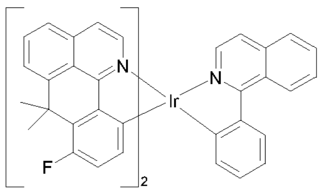
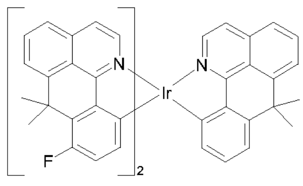

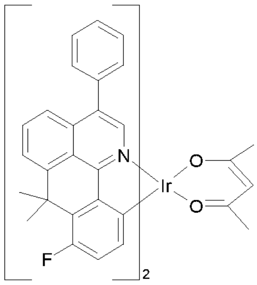
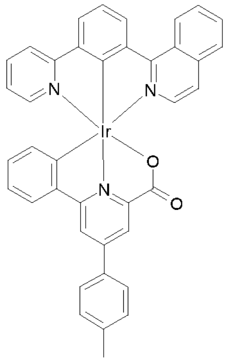
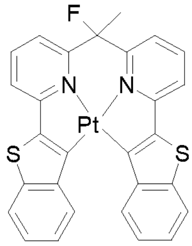

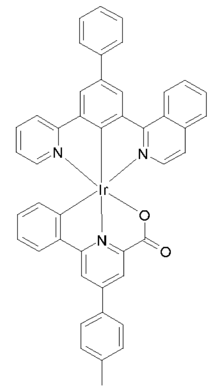

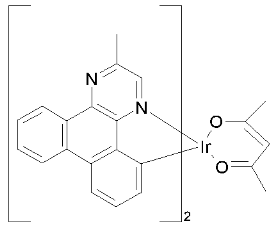

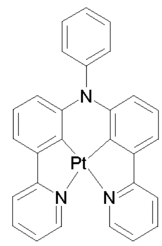

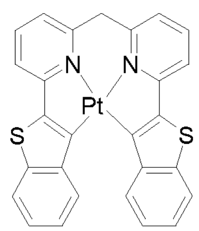

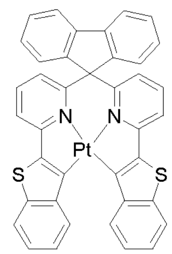

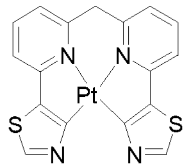

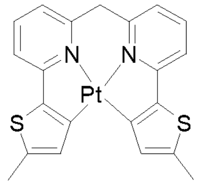

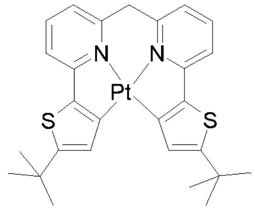

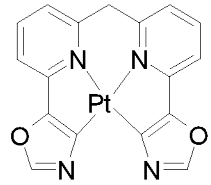

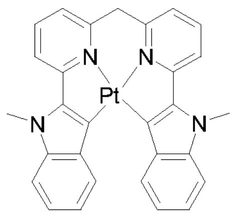

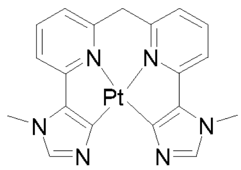

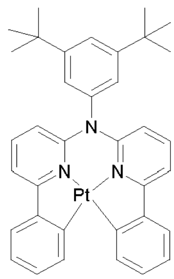

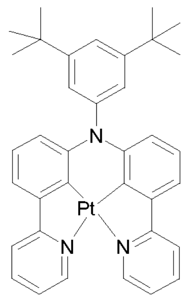

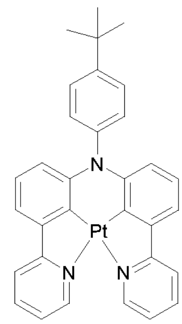

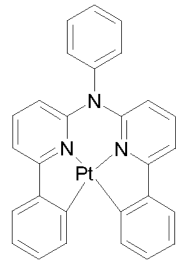

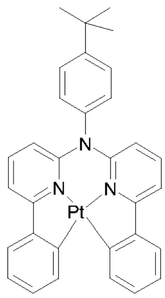
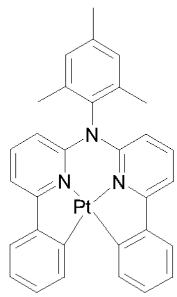
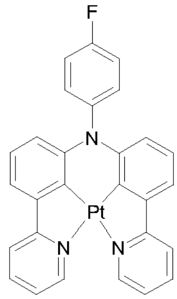

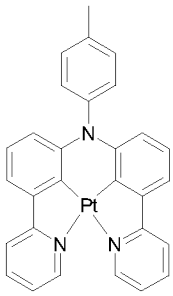
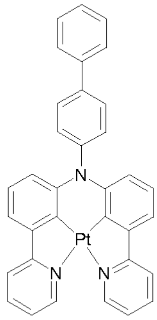
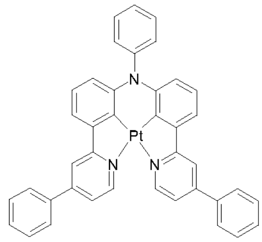

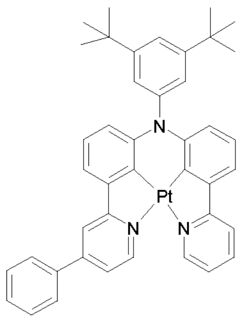
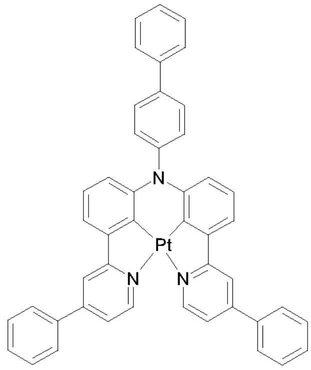
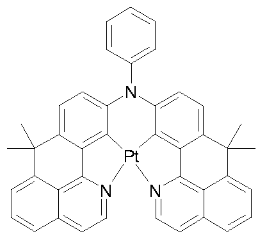

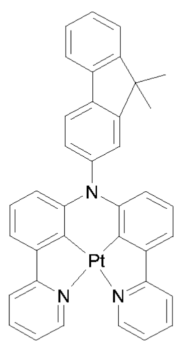
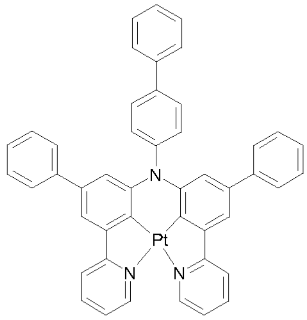
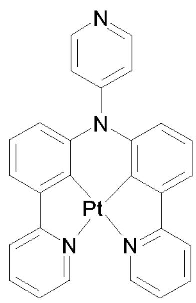

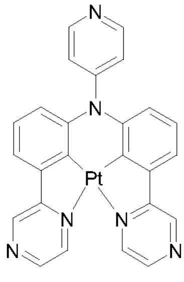
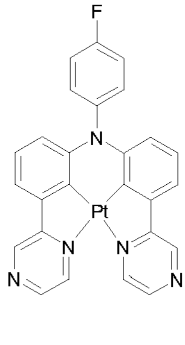
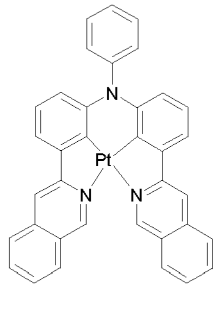

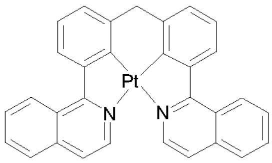

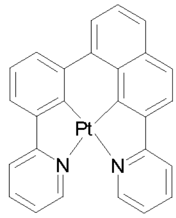

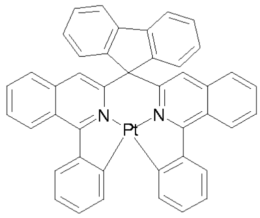

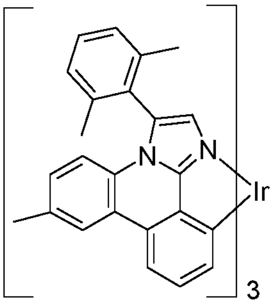

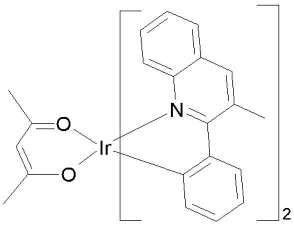

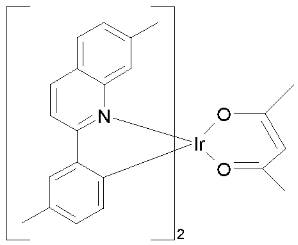

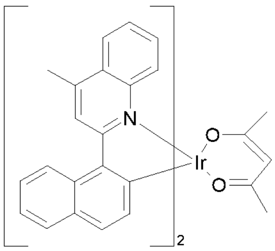

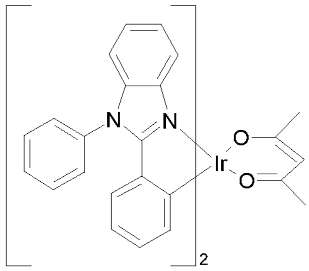

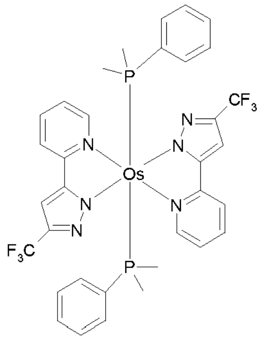

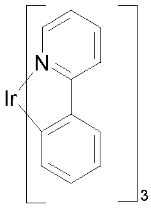

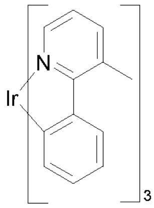

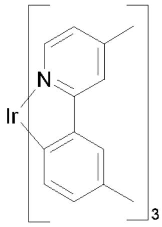

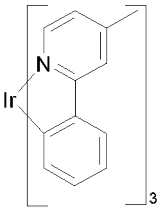

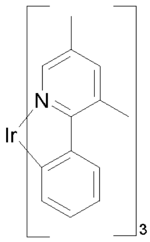

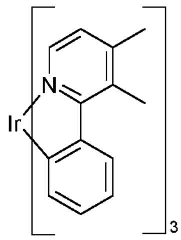

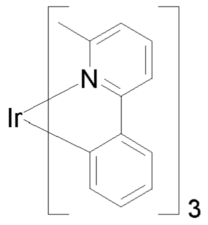

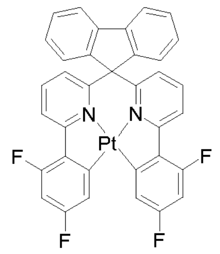

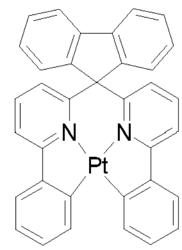

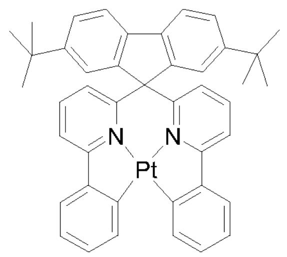

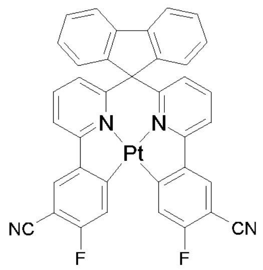

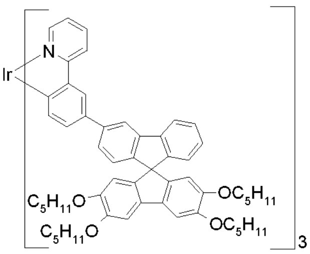

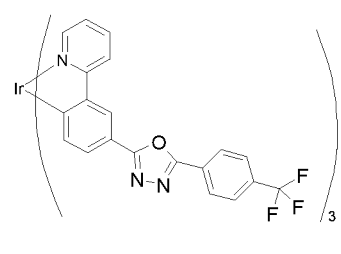

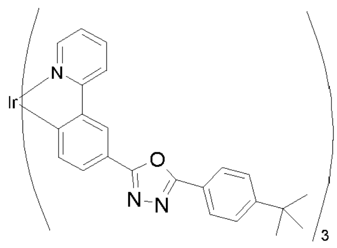

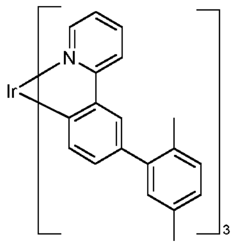

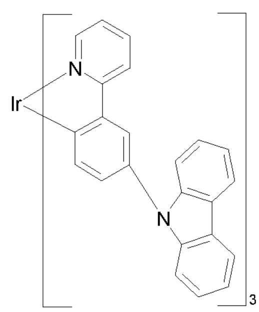

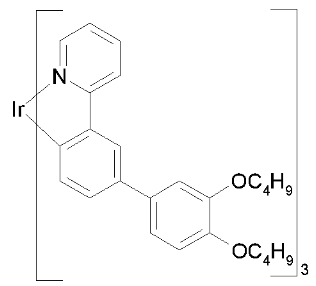

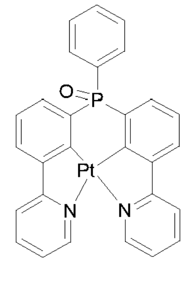

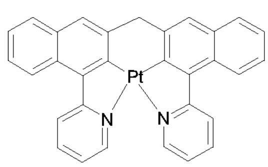

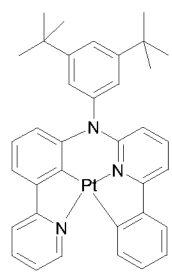

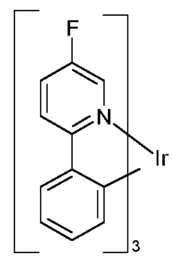

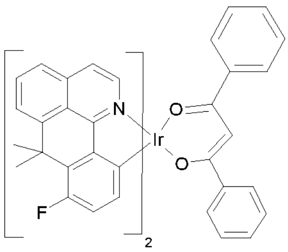

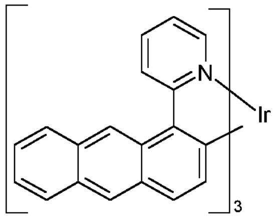

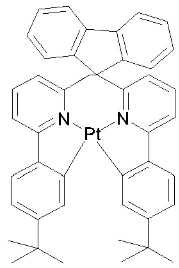

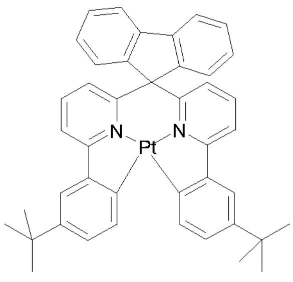

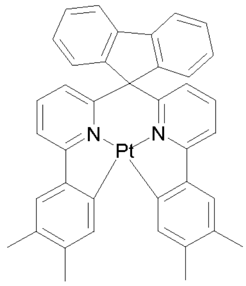

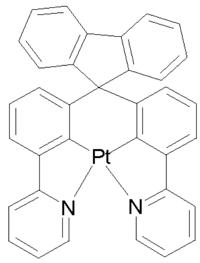

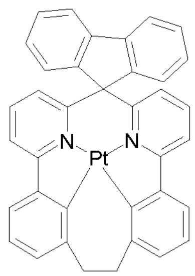

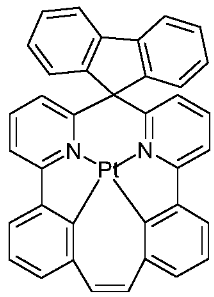

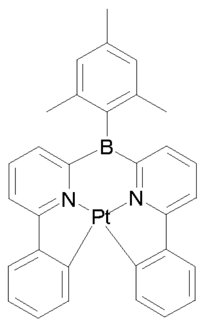

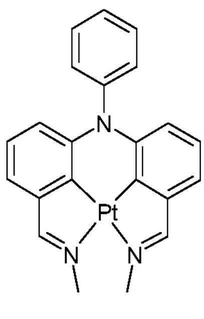

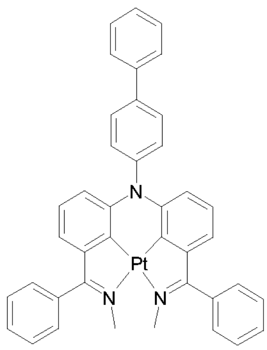

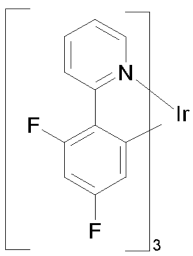

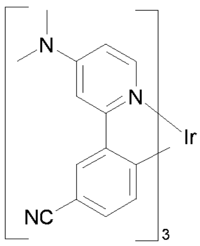

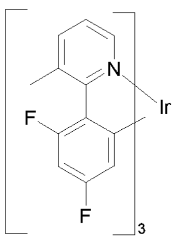

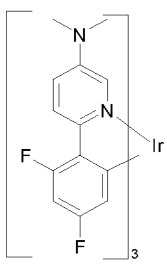

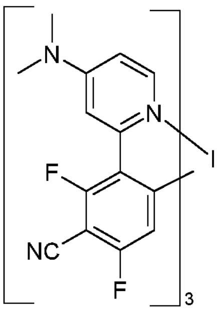
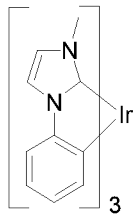
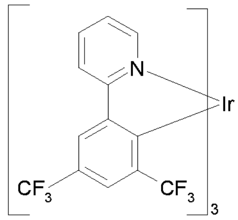

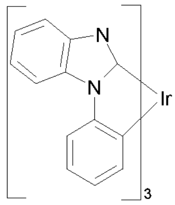
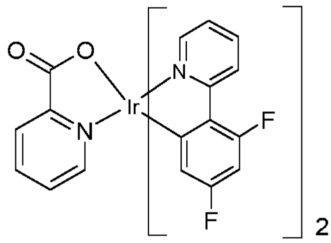
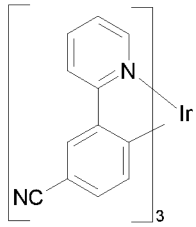

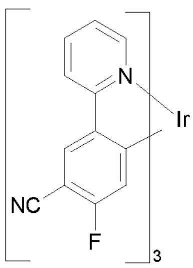
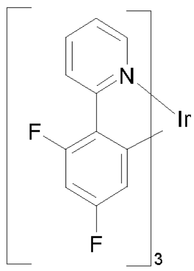
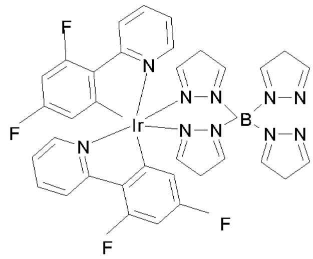

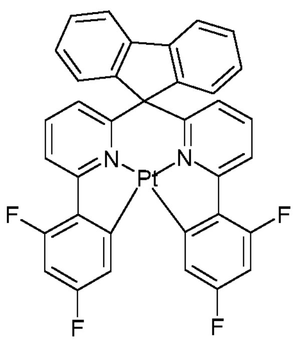
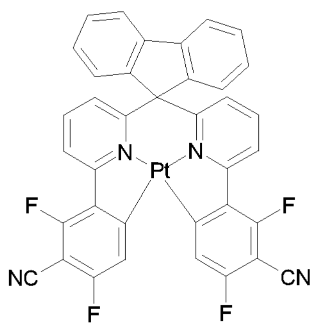
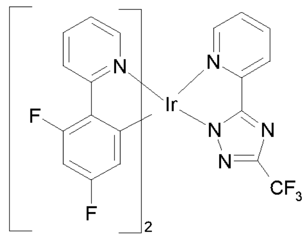

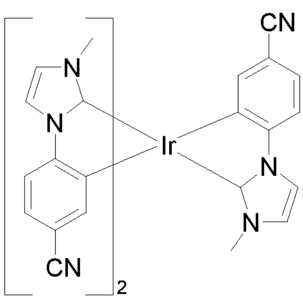
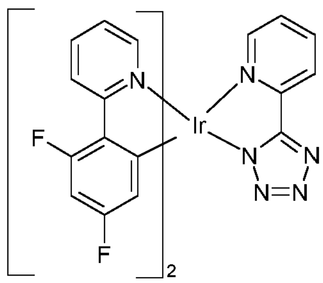
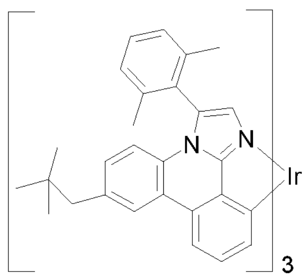

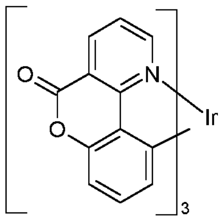
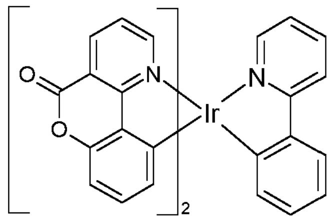
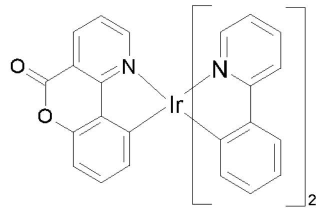

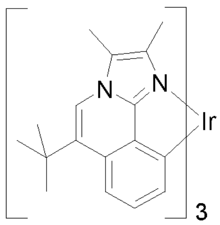
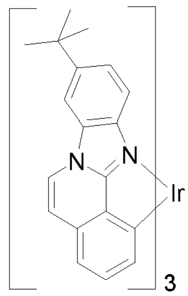
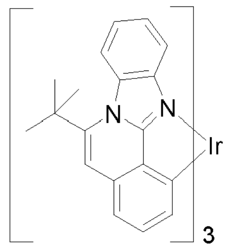

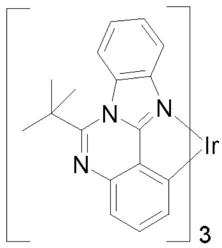
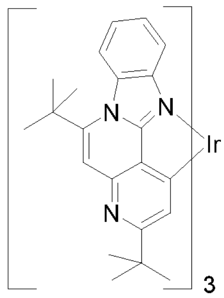
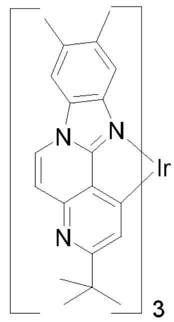

Die oben beschriebenen Verbindung, umfassend Strukturen der Formel (I), bzw. die oben aufgeführten bevorzugten Ausführungsformen können in einer elektronischen Vorrichtung bevorzugt als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung, umfassend Strukturen der Formel (I), bzw. die zuvor oder nachfolgend aufgeführten bevorzugten Ausführungsformen, enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs) oder organischen Laserdioden (O-Laser), enthaltend in mindestens einer Schicht mindestens eine Verbindung, umfassend Strukturen der Formeln (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser

Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. Eine emittierende Schicht umfasst mindestens eine emittierende Verbindung.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrix-materialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) bzw. die zuvor und nachfolgend aufgeführten bevorzugten Ausführungsformen in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) bzw. die zuvor und nachfolgend aufgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) bzw. die zuvor und nachfolgend aufgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden

Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als

Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formel (I) bzw. die zuvor und nachfolgend aufgeführten bevorzugten Ausführungsformen und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formel (I) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend Verbindungen mit Strukturen gemäß Formel (I) bzw. die zuvor aufgeführten bevorzugten Ausführungsformen, insbesondere als elektronenleitende und/oder als lochleitende Materialien, weisen eine sehr gute Lebensdauer auf.

2. Elektronischen Vorrichtungen, insbesondere organische Elektrolumineszenzvorrichtungen enthaltend Verbindungen mit Strukturen gemäß Formel (I) bzw. die zuvor aufgeführten bevorzugten Ausführungsformen als elektronenleitende und/oder als lochleitende Materialien weisen eine hervorragende Effizienz auf. Insbesondere ist die Effizienz deutlich höher gegenüber analogen Verbindungen, die keine Struktureinheit gemäß Formel (I) enthalten.

3. Die erfindungsgemäßen Verbindungen mit Strukturen gemäß Formel (I) bzw. die zuvor aufgeführten bevorzugten Ausführungsformen zeigen eine sehr hohe Stabilität und führen zu Verbindungen mit einer sehr hohen Lebensdauer.

4. Mit Verbindungen mit Strukturen gemäß Formel (I) bzw. die zuvor aufgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen mit Strukturen gemäß Formel (I) bzw. die zuvor aufgeführten bevorzugten Ausführungsformen in Schichten elektronischer Vorrichtungen, insbesondere organischer Elektrolumineszenzvorrichtungen führt zu einer hohen Mobilität der Elektron-Leiterstrukturen und/oder der Loch-Leiterstrukturen.

6. Verbindungen mit Strukturen gemäß Formel (I) bzw. die zuvor aufgeführten bevorzugten Ausführungsformen zeichnen sich durch eine ausgezeichnete thermische Stabilität aus, wobei Verbindungen mit einer Molmasse von weniger als ca. 1200 g/mol gut sublimierbar sind.

7. Verbindungen mit Strukturen gemäß Formel (I) bzw. die zuvor aufgeführten bevorzugten Ausführungsformen weisen eine ausgezeichnete Glasfilmbildung auf.

8. Verbindungen mit Strukturen gemäß Formel (I) bzw. die zuvor aufgeführten bevorzugten Ausführungsformen bilden aus Lösungen sehr gute Filme.

9. Die Verbindungen mit Strukturen gemäß Formel (I) bzw. die zuvor aufgeführten bevorzugten Ausführungsformen weisen ein überraschend hohes Triplett-Niveau $T_1$ auf, wobei dies insbesondere Verbindungen gilt, die als elektronenleitende Materialien eingesetzt werden.

Diese oben genannten Vorteile gehen nicht mit einer Verschlechterung der weiteren elektronischen Eigenschaften einher.

Ein weiterer Gegenstand der vorliegenden Erfindung ist die Verwendung einer erfindungsgemäßen Verbindung in einer elektronischen Vorrichtung als Lochblockiermaterial, Elektroneninjektionsmaterial und/oder Elektronentransportmaterial.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die mit der vorliegenden Erfindung offengelegte Lehre zum technischen Handeln kann abstrahiert und mit

anderen Beispielen kombiniert werden.

**Referenz-Beispiele:**

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

**Synthesebeispiele von alternativen**, nicht erfindungsgemässen **Verbindungen**

**a) 3-Dibenzofuran-4-yl-9H-carbazol**

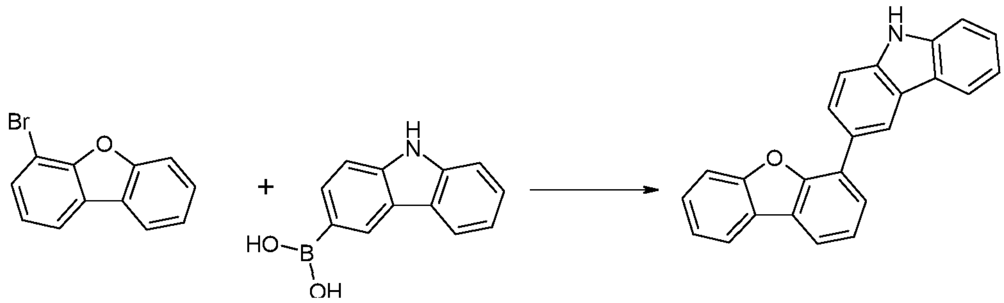

[851524-97-5 ]

6,75 g (32 mmol) *B-(9H-carbazol-3-yl)boronsäure,* 7,8 g (31,6 mmol) 4-Bromdibenzofuran, 31 ml (63 mmol) $Na_2CO_3$ (2 M-Lösung) werden in 120 mL Toulol, 120 mL Ethanol suspendiert. Zu dieser Suspension werden 0,73 g (0,63 mmol) $Pd(PPh_3)_4$ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 7,66 g (23 mmol), entsprechend 73% der Theorie

Analog können die folgenden Referenz-Verbindungen erhalten werden

| | Edukt 1 | Edukt 2 | Produkt | Ausbeute |
|---|---|---|---|---|
| a1 | 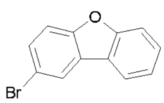 22439-61-8 ] | 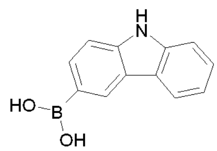 [851524-97-5 ] | 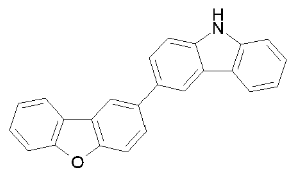 | 67% |
| a2 | 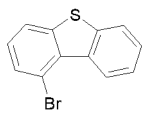 [65642-94-6 ] | 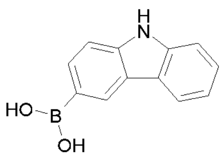 [851524-97-5 ] | 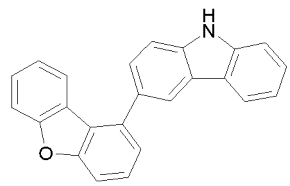 | 72% |

(fortgesetzt)

| | Edukt 1 | Edukt 2 | Produkt | Ausbeute |
|---|---|---|---|---|
| a3 | 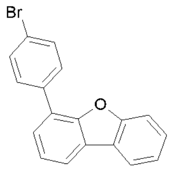 [955959-84-9 ] | 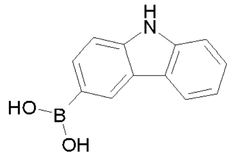 [851524-97-5 ] | 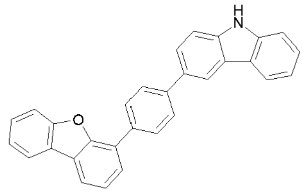 | 79% |
| a4 | 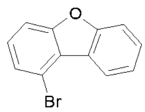 [50548-45-3 ] | 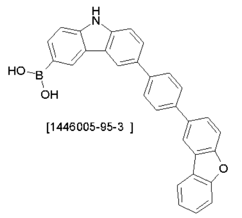 [1446005-95-3 ] | 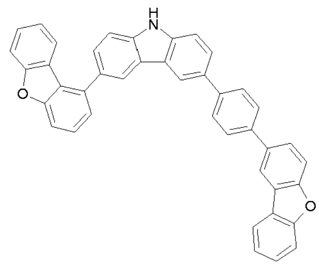 | 81% |
| a5 | 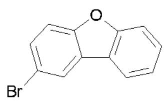 22439-61-8 ] | 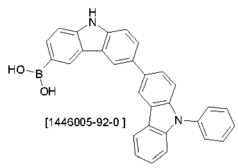 [1446005-92-0 ] | 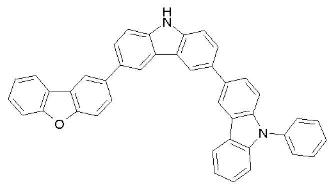 | 79% |
| a6 | 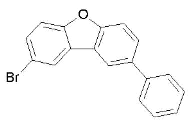 [1338446-67-5 ] | 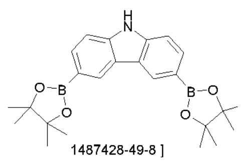 1487428-49-8 ] | 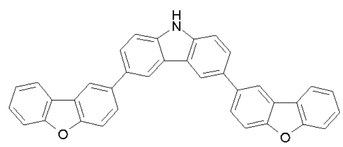 | 79% |
| a7 | 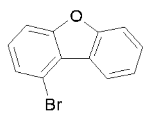 [50548-45-3 ] | 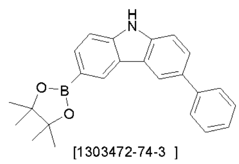 [1303472-74-3 ] | 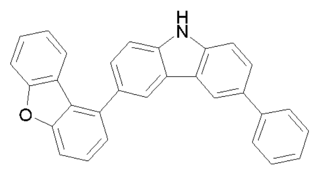 | 82% |
| a8 | 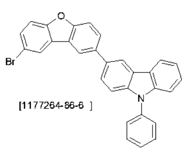 [1177264-86-6 ] | 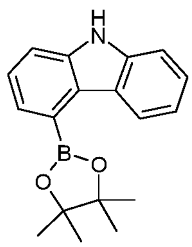 [1255309-13-7 ] | 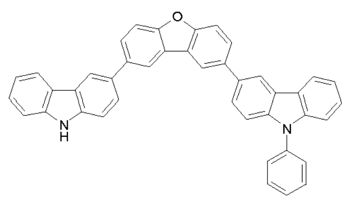 | 69% |
| a9 | 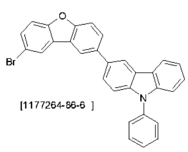 [1177264-86-6 ] | 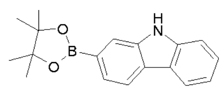 [1242412-60-7 ] | 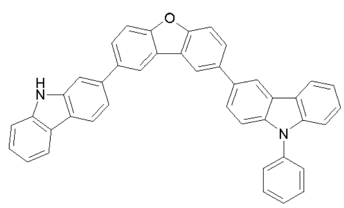 | 74% |

(fortgesetzt)

| | | | | |
|---|---|---|---|---|
| a10 | 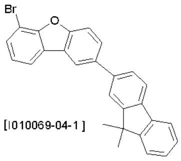 [1010069-04-1] | 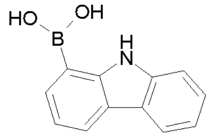 [869642-36-4] | 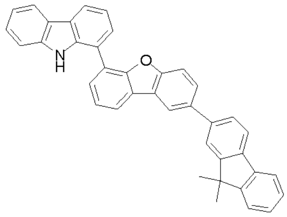 | 76% |
| a11 | 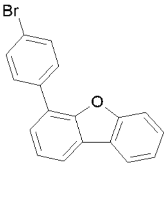 [955959-84-9] | 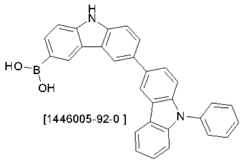 [1446005-92-0] | 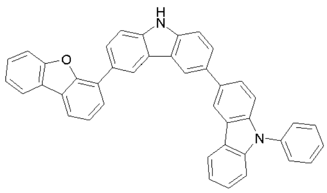 | 68% |
| a12 | 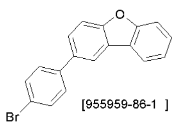 [955959-86-1] | 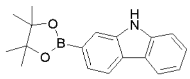 [1242412-60-7] | 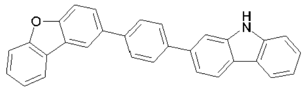 | 75% |
| a13 | 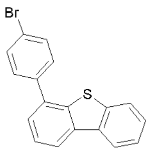 [530402-77-8] | 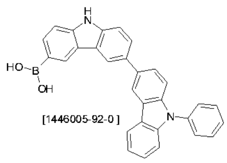 [1446005-92-0] | 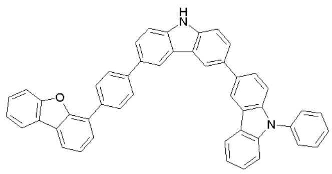 | 67% |
| a14 | 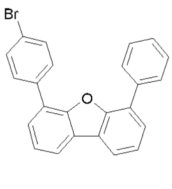 [1556069-46-5] | 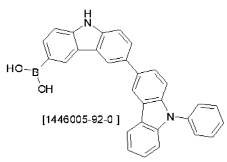 [1446005-92-0] | 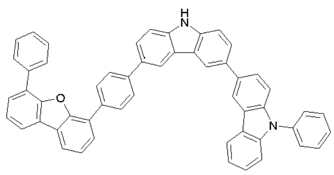 | 77% |
| a15 | 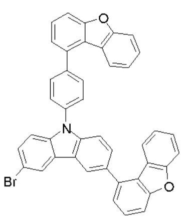 | 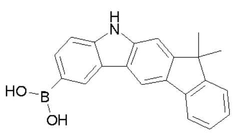 [1357286-77-1] | 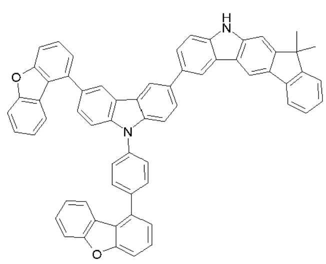 | 76% |

Bei den folgenden Verbindungen wird der Rückstand mit Toluol heiß extrahiert, aus Toluol umkristallisiert und abschließend im Hochvakuum sublimiert. HPLC Reinheit ist grösser 99,9%.

| | Edukt 1 | Edukt 2 | Produkt | Ausbeute |
|---|---|---|---|---|
| a16 | 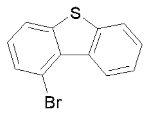 [65642-94-6 ] | 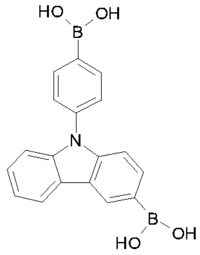 [1257324-73-4 ] | 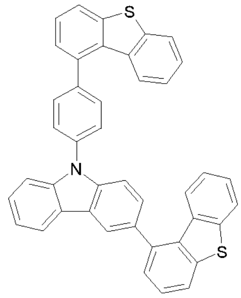 | 63% |
| a17 | 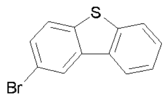 [82024-22-4 ] | 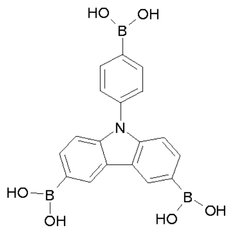 [1416368-44-9 ] | 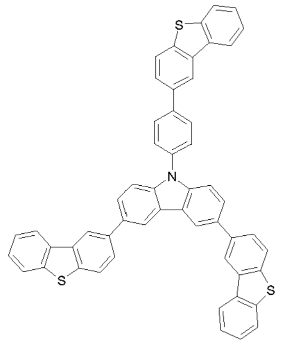 | 64% |
| a18 | 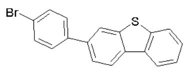 [1557258-01-1 ] | 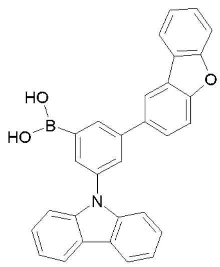 [1098258-06-0 ] | 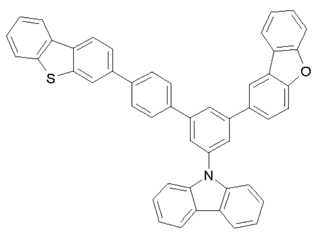 | 65% |
| a19 | 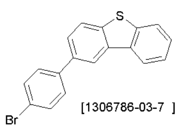 [1306786-03-7 ] | 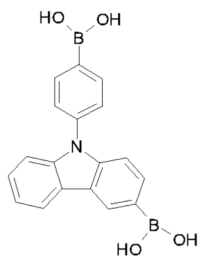 [1257324-73-4 ] | 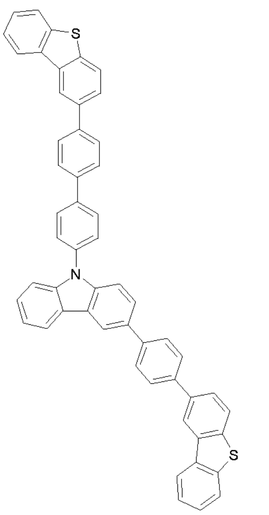 | 60% |

(fortgesetzt)

| | | | | |
|---|---|---|---|---|
| a20 | 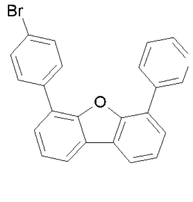 [1556069-46-5 ] | 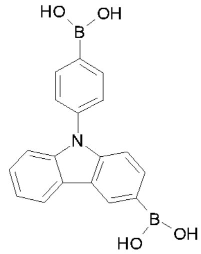 [1257324-73-4 ] | 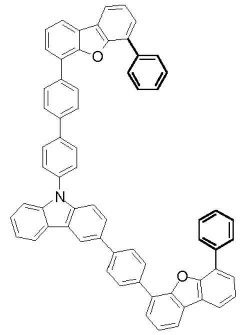 | 63% |

| | | | | |
|---|---|---|---|---|
| a21 | 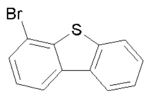 [97511-05-2 ] | 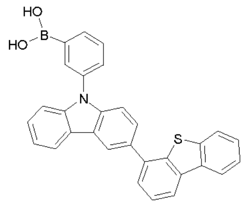 [1420067-45-3 ] | 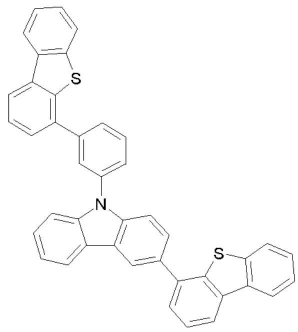 | 67% |
| a22 | 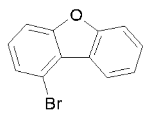 [50548-45-3 ] | 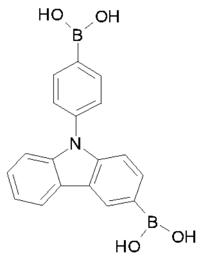 [1257324-73-4 ] | 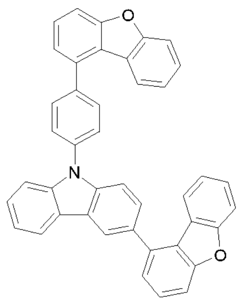 | 77% |

**b) 7-Bromo-10-(3-dibenzothiophen-4-yl-phenyl)-12,12-dimethyl-10,12-dihydro-10-aza-indeno[2,1 -b]fluorene**

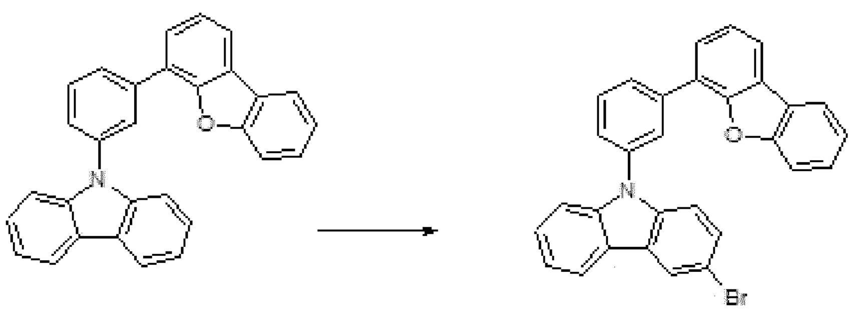

[1422393-46-1]

15,2 g (37,3mmol) 9-(3-Dibenzofuran-4-yl-phenyl)-9H-carbazole werden in 80 mL DMF vorgelegt. Anschließend werden

13,3 g (74,6 mmol) NBS in portionsweise zugegeben und rührt 4 h weiter bei dieser Temperatur. Anschließend wird die Mischung mit 15mL Wasser versetzt und mit $CH_2Cl_2$ extrahiert. Die organische Phase wird über $MgSO_4$ getrocknet und die Lösungsmittel im Vakuum entfernt. Das Produkt wird mit Hexan heiß ausgerührt und abgesaugt. Ausbeute: 14,6 g (29 mmol), 78 % der Theorie, Reinheit nach 1H NMR ca. 97 %.

| | Edukt 1 | Produkt | Ausbeute |
|---|---|---|---|
| b1 | 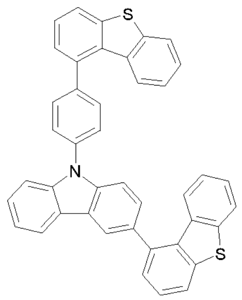 | 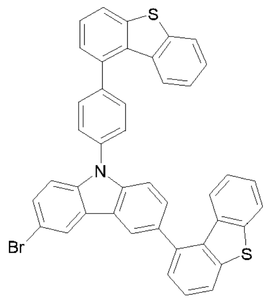 | 68% |
| b2 | 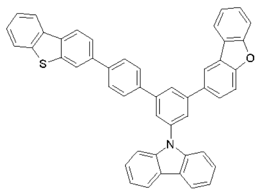 | 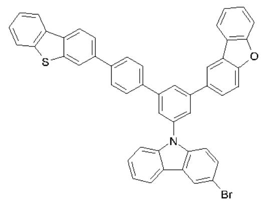 | 64% |
| b3 | 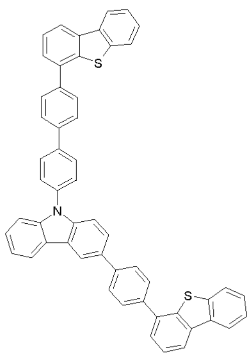 | 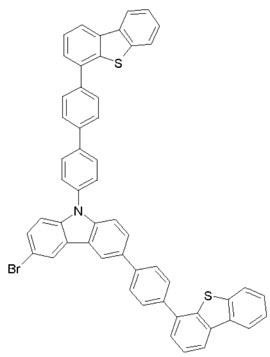 | 63% |
| b4 | 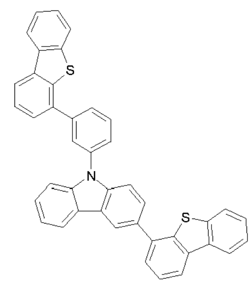 | 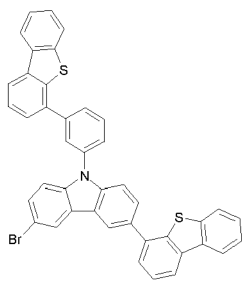 | 67% |
| b5 | 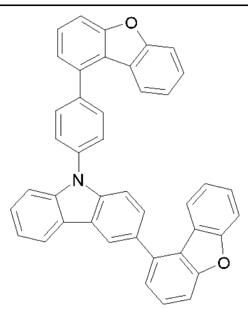 | 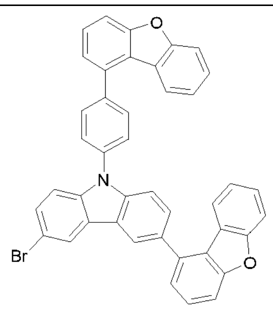 | 65% |

(fortgesetzt)

| | Edukt 1 | Produkt | Ausbeute |
|---|---|---|---|
| b6 | 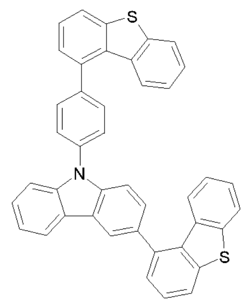 | 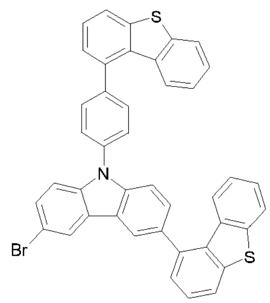 | 66% |

**c) 3-Dibenzofuran-4-yl-9-(4-dibenzofuran-4-yl-phenyl)-9H-carbazol**

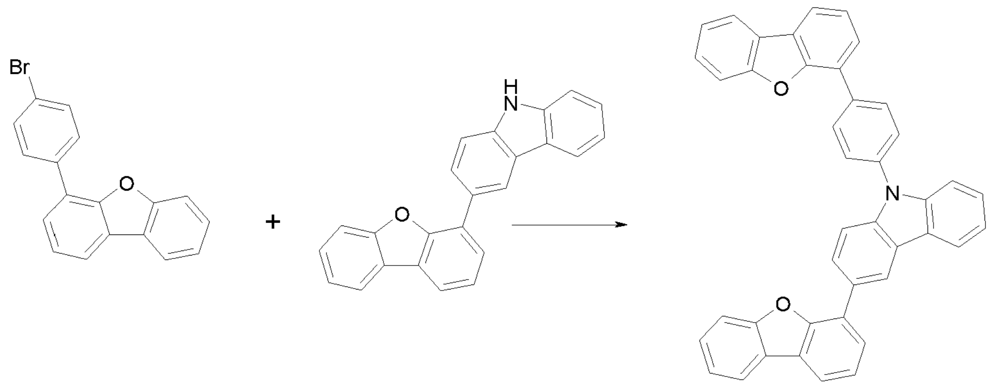

Unter Schutzgas werden 23,6 g (71 mmol) 3-(Dibenzofuran-4-yl)-9H-carbazol und 25 g (74 mmol) 4-(4-Bromophenyl)-dibenzofuran, 8g (84 mmol) Natrium-tert-Butylat , 3,5 ml Tris-tert-butyl-phosphin (1M in Toluol), 0,393 mg (1,7 mmol) Palladiumacetat in 300 ml p-Xylol suspendiert. Die Reaktionsmischung wird 12 h unter Rückfluss bei 110 °C erhitzt. Nach Erkalten wird die organische Phase abgetrennt, dreimal mit 200 mL Wasser gewaschen und anschließend zur Trockene eingeengt. Das Produkt wird via Säulenchromatographie an Kieselgel mit Toluol/Heptan (1:2) gereinigt. Der Rückstand wird mit Toluol heiß extrahiert, aus Toluol umkristallisiert und abschließend im Hochvakuum sublimiert. HPLC Reinheit ist grösser 99,9%.

Die Ausbeute beträgt 33,7 g (658 mmol), 80 % der Theorie, Reinheit nach $^{1}$H-NMR ca. 94 %.

Analog werden folgende Referenz-Verbindungen hergestellt:

| | Edukt 1 | Edukt 2 | Produkt | Ausbeute |
|---|---|---|---|---|
| C1 | 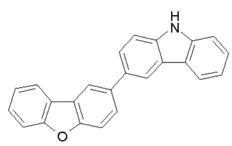 | 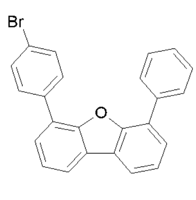 [1556069-46-5 ] | 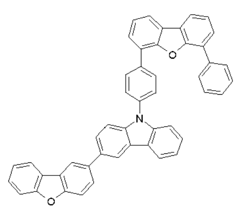 | 85% |

(fortgesetzt)

| | Edukt 1 | Edukt 2 | Produkt | Ausbeute |
|---|---|---|---|---|
| C2 | 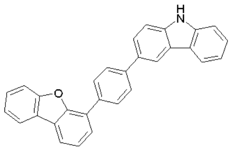 | 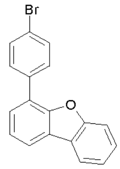 [955959-84-9 ] | 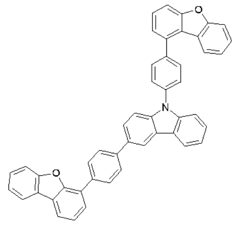 | 80% |
| C3 | 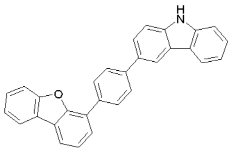 | 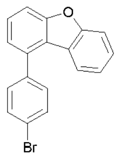 [1786416-89-4 ] | 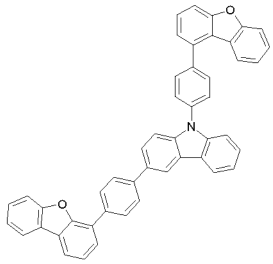 | 79% |
| C4 | 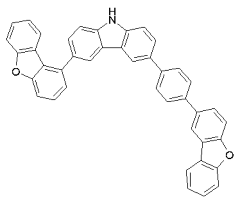 | 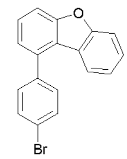 [1786416-89-4 ] | 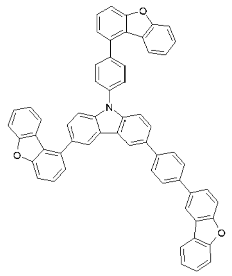 | 77% |
| C5 | 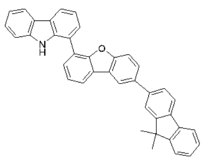 | 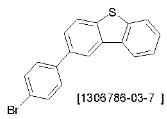 [1306786-03-7 ] | 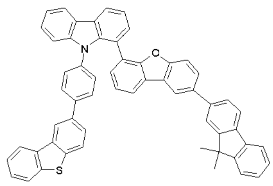 | 88% |
| C6 | 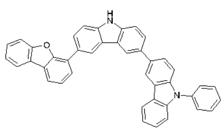 | 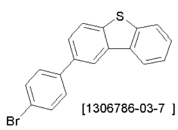 [1306786-03-7 ] | 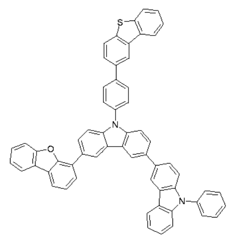 | 82% |
| C7 | 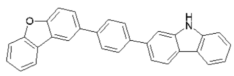 | 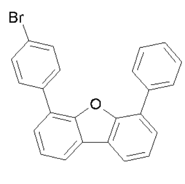 [1556069-46-5 ] | 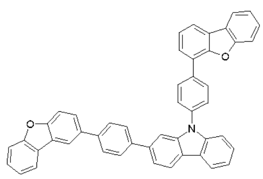 | 81% |

(fortgesetzt)

| | | | | |
|---|---|---|---|---|
| C8 | 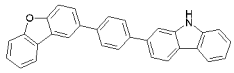 | 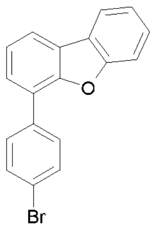 [955959-84-9 ] | 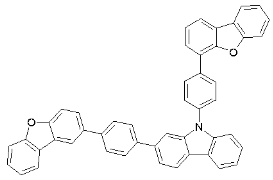 | 75% |
| C9 | 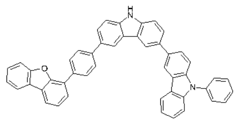 | 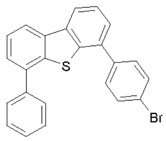 [1357081-21-0 ] | 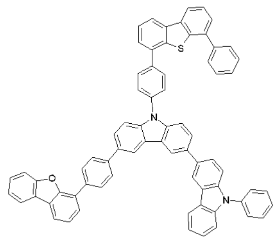 | 84% |

| | | | | |
|---|---|---|---|---|
| C10 | 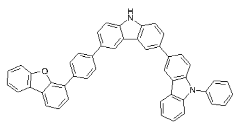 | 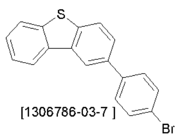 [1306786-03-7 ] | 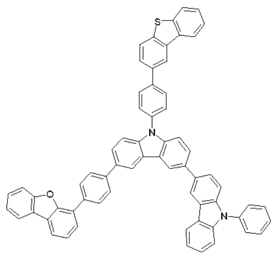 | 76% |
| C11 | 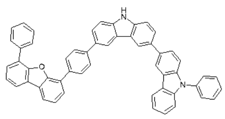 | 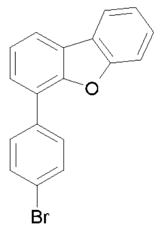 [955959-84-9 ] | 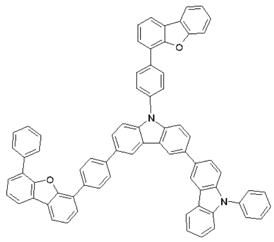 | 69% |
| C12 | 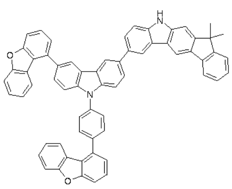 | 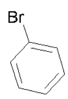 | 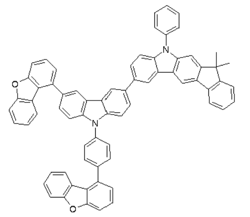 | 73% |

Analog zu Synthese von A9 können folgende Referenz-Verbindungen hergestellt werden:

| | Edukt 1 | Edukt 2 | Produkt | Ausbeute |
|---|---|---|---|---|
| C13 | 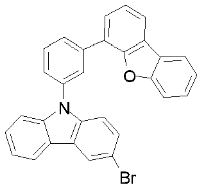 | 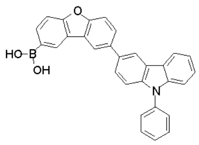 | 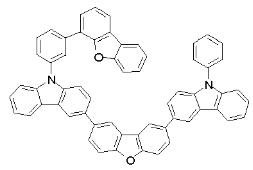 | 71% |
| C14 | 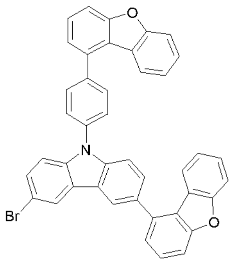 | 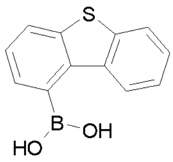 [1245943-60-5 ] | 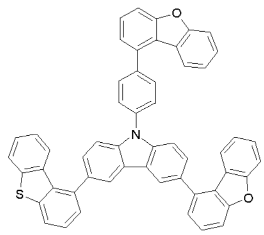 | 73% |
| C15 | 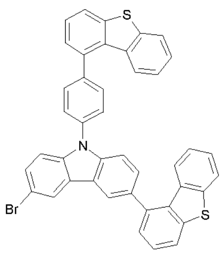 | 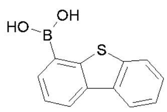 [108847-20-7 ] | 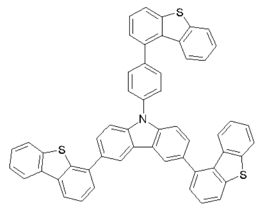 | 74% |
| C16 | 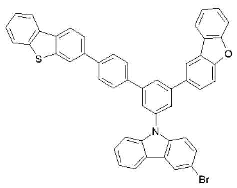 | 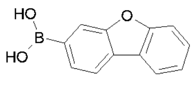 [056113-50-8 ] | 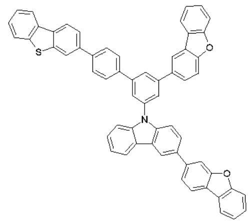 | 72% |
| C17 | 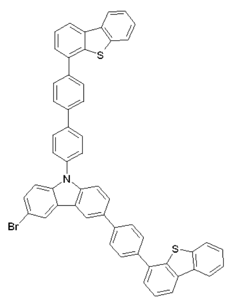 | 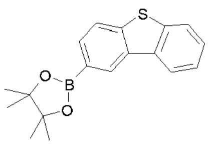 [890042-21-4 ] | 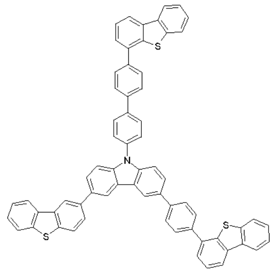 | 70% |
| C18 | 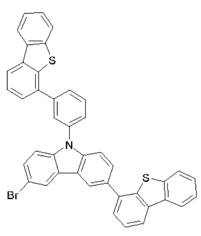 | 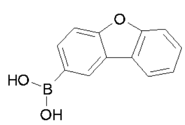 [402936-15-6 ] | 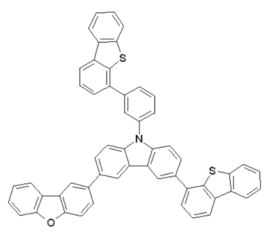 | 65% |

(fortgesetzt)

| | Edukt 1 | Edukt 2 | Produkt | Ausbeute |
|---|---|---|---|---|
| C19 | 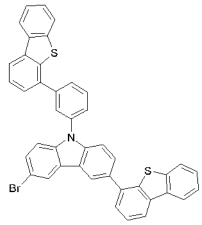 | 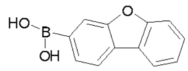 395087-89-5 ] | 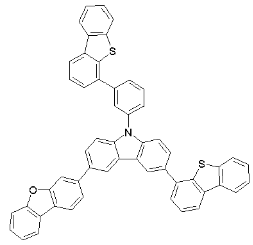 | 72% |
| C20 | 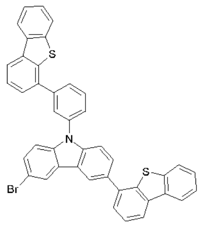 | 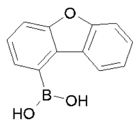 [162607-19-4 ] | 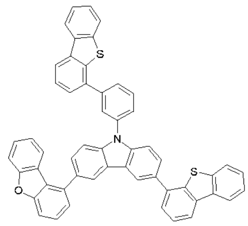 | 75% |
| C21 | 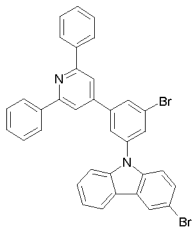 | 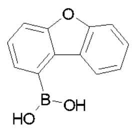 [162607-19-4 ] | 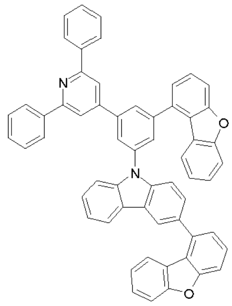 | 78% |
| C22 | 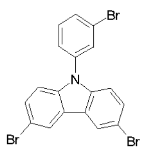 [865596-55-0 ] | 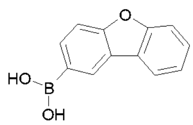 [402936-15-6 ] | 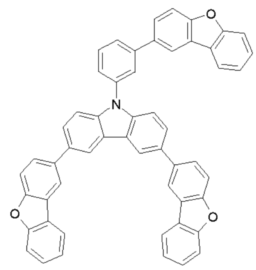 | 81% |
| C23 | 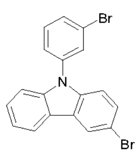 [1345415-05-5 ] | 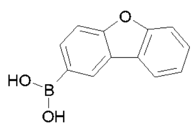 [402936-15-6 ] | 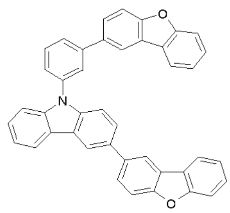 | 82% |
| C24 | 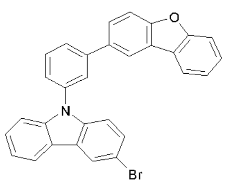 [1476799-03-7 ] | 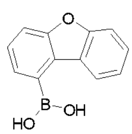 [162607-19-4 ] | 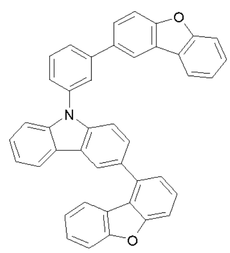 | 84% |

(fortgesetzt)

| | Edukt 1 | Edukt 2 | Produkt | Ausbeute |
|---|---|---|---|---|
| C25 | 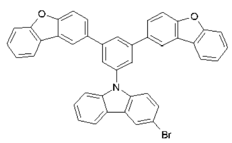 | 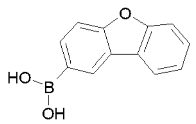 [402936-15-6 ] | 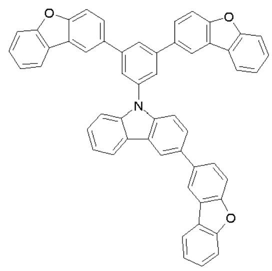 | 81% |
| C27 | 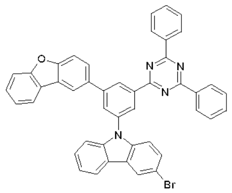 | 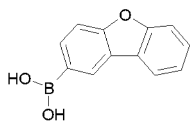 [402936-15-6 ] | 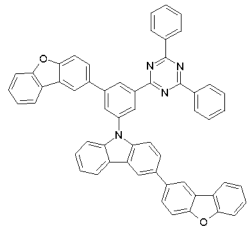 | 72% |

**Herstellung der OLEDs**

Die Herstellung von alternativen, nicht erfindungsgemässen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E10 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Optionale Lochinjektionsschicht (HIL) / Lochtransportschicht (HTL) / Optionale Zwischenschicht (IL) / Elektronenblockierschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie ST1:CBP:TER1 (55%:35%:10%) bedeutet hierbei, dass das Material ST1 in einem Volumenanteil von 55%, CBP in einem Anteil von 35% und TER1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 bezeichnet die Stromeffizienz, die bei 1000 cd/$m^2$ erreicht werden.

Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/$m^2$ und L1 = 80% in Tabelle 2 bedeutet, dass die in Spalte LD angegebene Lebensdauer der Zeit entspricht, nach der die Anfangsleuchtdichte von 4000 cd/$m^2$ auf 3200 cd/$m^2$ absinkt. Analog bedeutet L0;j0 = 20mA/$cm^2$, L1 = 80%, dass die anfängliche Leuchtdichte bei Betrieb mit 20mA/$cm^2$ nach der Zeit LD auf 80% ihres Anfangswertes absinkt.

Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von

1000 cd/m$^2$ eine übliche Angabe.

Die Daten der verschiedenen OLEDs sind in Tabelle 2 zusammengefasst.

Die Beispiel V1-V6 sind Vergleichsbeispiele gemäß dem Stand der Technik, die Beispiele E1-E10 zeigen Daten von OLEDs mit alternativen und ebenfalls nicht erfindungsgemässen Materialien.

Tabelle 1: Aufbau der OLEDs

| Bsp | HTL Dicke | IL Dicke | EBL Dicke | EML Dicke | HBL Dicke | ETL Dicke | EIL Dick e |
|---|---|---|---|---|---|---|---|
| V1 | SpA1 70nm | HATCN 5nm | SpMA1 90nm | SdT1:IC1:TEG1 (45%: 45%:10%) 30nm | ST2 10nm | ST2:LiQ (50%: 50%) 30nm | --- |
| V2 | SpA1 70nm | HATCN 5nm | SpMA1 90nm | SdT2:IC1:TEG1 (45%: 45%:10%) 30nm | ST2 10nm | ST2:LiQ (50%: 50%) 30nm | --- |
| V3 | SpA1 70nm | HATCN 5nm | SpMA1 90nm | SdT3:IC1:TEG1 (45%: 45%:10%) 30nm | ST2 10nm | ST2:LiQ (50%: 50%) 30nm | --- |
| V4 | SpA1 70nm | HATCN 5nm | SpMA1 90nm | SdT4:IC1:TEG1 (45%: 45%:10%) 30nm | ST2 10nm | ST2:LiQ (50%: 50%) 30nm | --- |
| V5 | SpA1 70nm | HATCN 5nm | SpMA1 90nm | SdT5:IC1:TEG1 (45%: 45%:10%) 30nm | ST2 10nm | ST2:LiQ (50%: 50%) 30nm | --- |
| V6 | SpA1 70nm | HATCN 5nm | SpMA1 90nm | SdT6:IC1:TEG1 (45%: 45%:10%) 30nm | ST2 10nm | ST2:LiQ (50%: 50%) 30nm | --- |
| V7 | SpA1 70nm | HATCN 5nm | SpMA1 90nm | SdT7:IC1:TEG1 (45%: 45%:10%) 30nm | ST2 10nm | ST2:LiQ (50%: 50%) 30nm | --- |
| E1 | SpA1 70nm | HATCN 5nm | SpMA1 90nm | EG1:IC1:TEG1 (45%: 45%:10%) 30nm | ST2 10nm | ST2:LiQ (50%: 50%) 30nm | --- |
| E2 | SpA1 70nm | HATCN 5nm | SpMA1 90nm | EG2:IC1:TEG1 (45%: 45%:10%) 30nm | ST2 10nm | ST2:LiQ (50%: 50%) 30nm | --- |
| E3 | SpA1 70nm | HATCN 5nm | SpMA1 90nm | EG3:IC1:TEG1 (45%: 45%:10%) 30nm | ST2 10nm | ST2:LiQ (50%: 50%) 30nm | --- |
| E4 | SpA1 70nm | HATCN 5nm | SpMA1 90nm | EG4:IC1:TEG1 (45%: 45%:10%) 30nm | ST2 10nm | ST2:LiQ (50%: 50%) 30nm | --- |
| E1-1 | SpA1 90nm | HATCN 5nm | SpMA1 130nm | EG1:IC1:TER1 (32%: 60%:8%) 30nm | --- | ST2:LiQ (50%: 50%) 40nm | --- |
| E5 | SpA1 70nm | HATCN 5nm | SpMA1 90nm | EG5:IC1:TEG1 (45%: 45%:10%) 30nm | ST2 10nm | ST2:LiQ (50%: 50%) 30nm | --- |
| E7 | SpA1 70nm | HATCN 5nm | SpMA1 90nm | EG7:IC1:TEG1 (45%: 45%:10%) 30nm | ST2 10nm | ST2:LiQ (50%: 50%) 30nm | --- |
| E8 | SpA1 70nm | HATCN 5nm | SpMA1 90nm | EG8:IC1:TEG1 (45%: 45%:10%) 30nm | ST2 10nm | ST2:LiQ (50%: 50%) 30nm | --- |
| E9 | SpA1 70nm | HATCN 5nm | SpMA1 90nm | EG9:IC1:TEG1 (45%: 45%:10%) 30nm | ST2 10nm | ST2:LiQ (50%: 50%) 30nm | --- |
| E10 | HATCN 5nm | SpMA1 70nm | SpMA2 15nm | EG8:L1:TEY1 (45%: 45%:10%) 25nm | --- | ST1 45nm | LiQ 3nm |

Tabelle 2: Daten der OLEDs

| Bsp. | U1000 (V) | SE1000 (cd/A) | CIE x/y bei 1000 cd/m$^2$ | $L_0$; $j_0$ | L1 % | LD (h) |
|---|---|---|---|---|---|---|
| V1 | 3.9 | 55 | 0.33/0.63 | 20mA/cm$^2$ | 80 | 290 |
| V2 | 3.6 | 57 | 0.33/0.63 | 20mA/cm$^2$ | 80 | 250 |

(fortgesetzt)

| Bsp. | U1000 (V) | SE1000 (cd/A) | CIE x/y bei 1000 cd/m$^2$ | $L_0$; $j_0$ | L1 % | LD (h) |
|---|---|---|---|---|---|---|
| V3 | 3.9 | 55 | 0.33/0.63 | 20mA/cm$^2$ | 80 | 230 |
| V4 | 4.1 | 54 | 0.32/0.63 | 20mA/cm$^2$ | 80 | 200 |
| V5 | 3.8 | 52 | 0.33/0.63 | 20mA/cm$^2$ | 80 | 150 |
| V6 | 3.6 | 55 | 0.33/0.63 | 20mA/cm$^2$ | 80 | 190 |
| V7 | 3.5 | 57 | 0.33/0.63 | 20mA/cm$^2$ | 80 | 300 |
| E1 | 3.5 | 60 | 0.33/0.62 | 20mA/cm$^2$ | 80 | 350 |
| E2 | 3.8 | 58 | 0.33/0.62 | 20mA/cm$^2$ | 80 | 280 |
| E3 | 4.1 | 55 | 0.33/0.63 | 20mA/cm$^2$ | 80 | 230 |
| E4 | 3.7 | 54 | 0.32/0.63 | 20mA/cm$^2$ | 80 | 230 |
| E1-1 | 4.5 | 11.5 | 0.67/0.34 | 4000 cd/m$^2$ | 80 | 390 |
| E5 | 3.6 | 58 | 0.33/0.63 | 20mA/cm$^2$ | 80 | 350 |
| E7 | 3.5 | 57 | 0.33/0.63 | 20mA/cm$^2$ | 80 | 340 |
| E8 | 3.7 | 59 | 0.33/0.63 | 20mA/cm$^2$ | 80 | 380 |
| E9 | 3.6 | 58 | 0.33/0.63 | 20mA/cm$^2$ | 80 | 370 |
| E10 | 3.0 | 77 | 0.44/0.55 | 50mA/cm$^2$ | 90 | 75 |

Tabelle 3: Strukturformeln der Materialien für die OLEDs

| | |
|---|---|
| 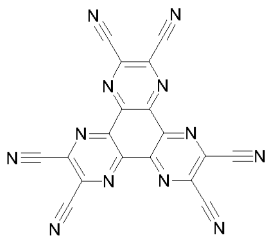 | 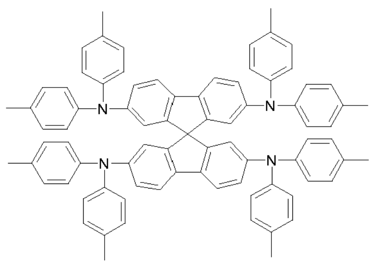 |
| HATCN | SpA1 |
| 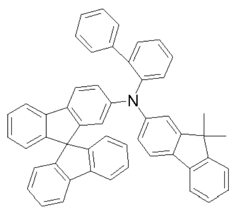 | 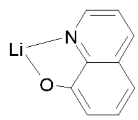 |
| SpMA1 | LiQ |
| 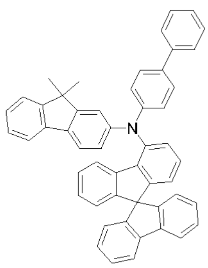 | 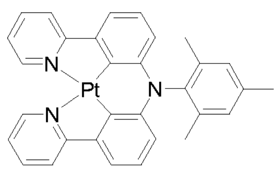 |
| SpMA2 | TER1 |

(fortgesetzt)

| | |
|---|---|
| 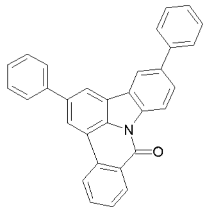 | 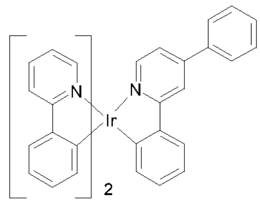 |
| L1 | TEY1 |
| 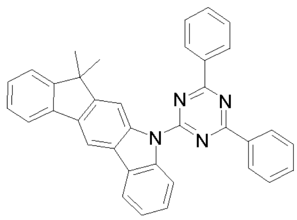 | 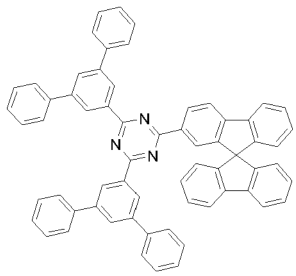 |
| IC1 | ST2 |
| | 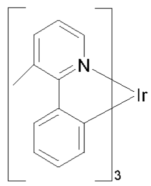 |
| | TEG1 |
| 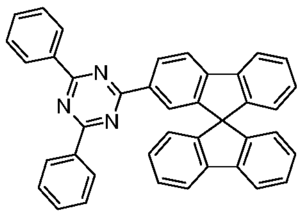 | 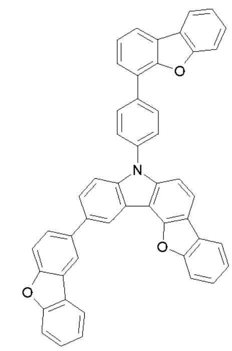 |
| ST1 | SdT1 |
| 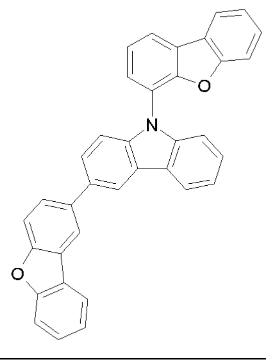 | 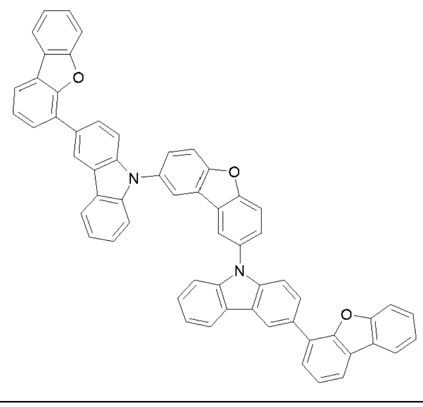 |
| SdT2 | SdT3 |

(fortgesetzt)

| | |
|---|---|
| 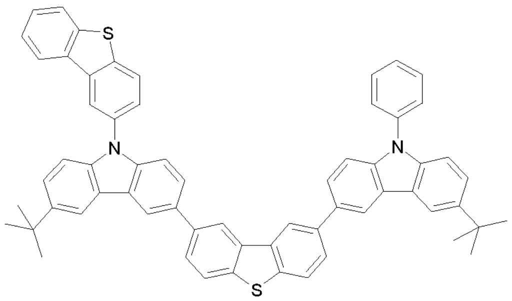 | 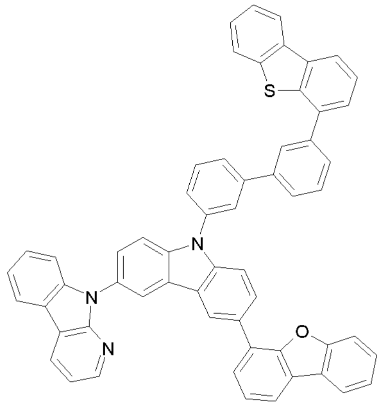 |
| SdT4 | SdT5 |
| 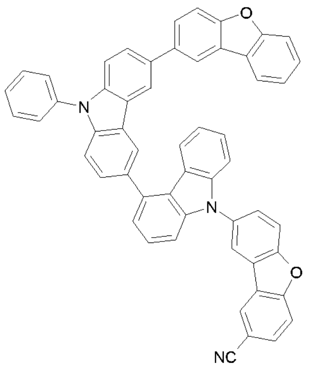 | 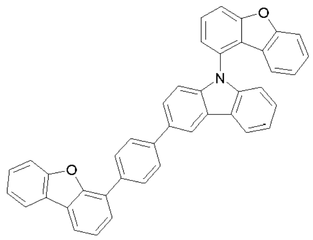 |
| SdT6 | SdT7 |
| 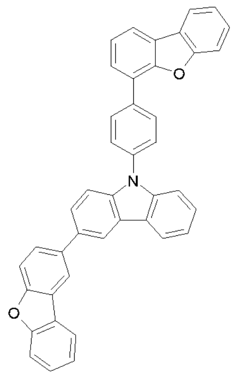 | 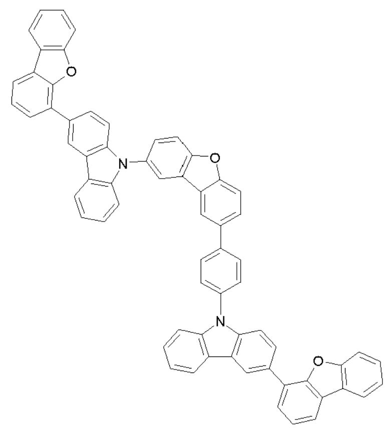 |
| EG1 | EG2 |

(fortgesetzt)

| | |
|---|---|
| 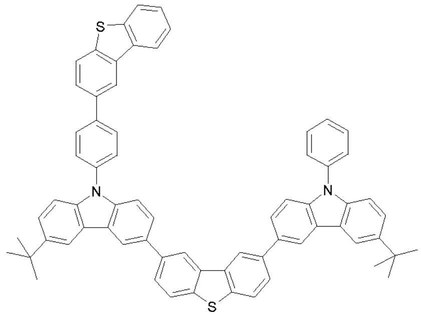 | 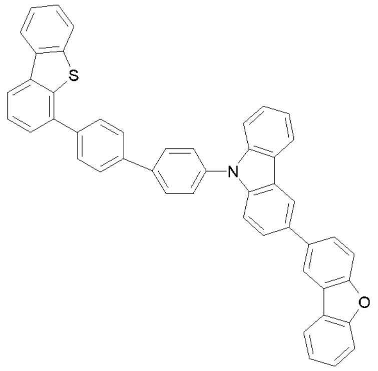 |
| EG3 | EG4 |
| 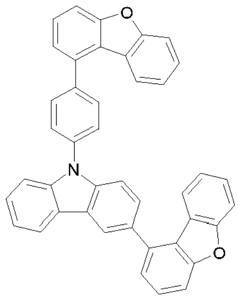 | |
| EG5 | |
| 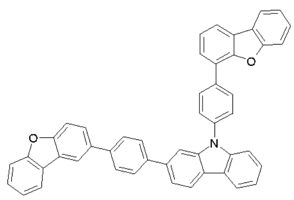 | 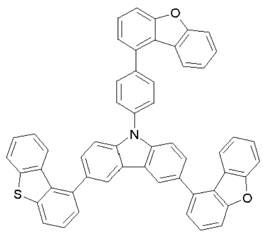 |
| EG7 | EG8 |
| 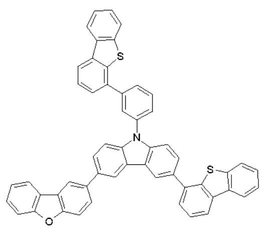 | |
| EG9 | |

wobei die alternativen Verbindungen EG1 bis EG9 ganz analog zu den oben genannten Verbindungen hergestellt werden können.

**Patentansprüche**

**1.** Verbindung, umfassend Strukturen der Formel (I),

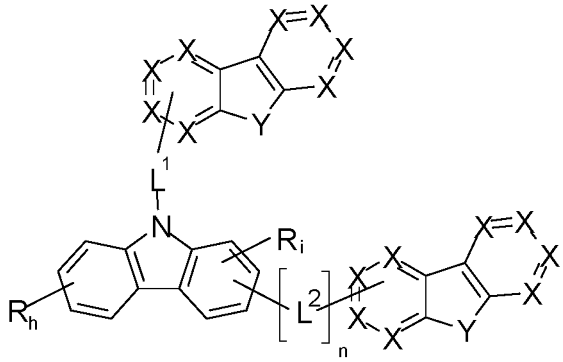

Formel (I),

wobei für die verwendeten Symbole gilt:

X ist $CR^1$ oder C für die Anbindungsstelle der Reste $L^1$, $L^2$ oder der Carbazolgruppe;
Y ist bei jedem Auftreten gleich oder verschieden O oder S;
$L^1$, $L^2$ ist ein aromatisches Ringsystem mit 6 bis 40 C-Atomen, das keine kondensierten aromatischen Ringe aufweist, wobei das aromatische Ringsystem durch einen oder mehrere Reste $R^4$ substituiert sein kann, wobei die Substituenten $R^4$ ebenfalls keine kondensierten aromatischen Ringe umfassen und ebenfalls keine heteroaromatischen Ringsysteme oder Heteroarylgruppen umfassen;

einer der Reste R ist eine Carbazolgruppe, die nicht über den Stickstoff der Carbazolgruppe gebunden ist und die jeweils durch einen oder mehrere Reste $R^2$ substituiert sein kann, und alle anderen Reste R sind gleich H;

$R^1$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^2)_3$, $B(OR^2)_2$, $OSO_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $P(=O)(R^2)$, SO, $SO_2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;
$R^2$ ist bei jedem Auftreten gleich oder verschieden H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch C=C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;
$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können auch zwei Reste $Ar^1$, welche an dasselbe Phosphoratom binden, durch eine Einfachbindung oder eine Brücke, ausgewählt aus $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ und $P(=O)R^3$, miteinander verknüpft sein;
$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

$R^4$ ist bei jedem Auftreten gleich oder verschieden H, D, F, Cl, Br, I, CHO, $C(=O)Ar^2$, $P(=O)(Ar^2)_2$, $S(=O)Ar^2$, $S(=O)_2Ar^2$, CN, $NO_2$, $Si(R^5)_3$, $B(OR^5)_2$, $OSO_2R^5$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch C=C , $Si(R^5)_2$, $Ge(R^5)_2$, $Sn(R^5)_2$, C=O, C=S, C=Se, $P(=O)(R^5)$, SO, $SO_2$, O, S oder $CONR^5$ ersetzt sein können und wobei ein oder mehrere H-Atome durch D, F, Cl, Br, I, CN oder $NO_2$ ersetzt sein können, oder ein aromatisches Ringsystem mit 5 bis 40 C-Atomen, das keine kondensierten aromatischen Ringe aufweist und das jeweils durch einen oder mehrere Reste $R^5$ substituiert sein kann, oder eine Aryloxygruppe mit 5 bis 40 aromatischen Ringatomen, die durch einen oder mehrere Reste $R^5$ substituiert sein kann, oder eine Kombination dieser Systeme, dabei können zwei oder mehrere benachbarte Substituenten $R^4$ auch miteinander ein mono- oder polycyclisches, aliphatisches Ringsystem bilden, das jedoch keine kondensierten aromatischen Ringe aufweist;

$Ar^2$ ist bei jedem Auftreten gleich oder verschieden ein aromatisches Ringsystem mit 5 bis 30 C-Atomen, das keine kondensierten aromatischen Ringe aufweist und das mit einem oder mehreren Resten $R^5$ substituiert sein kann;

$R^5$ ist bei jedem Auftreten gleich oder verschieden H, D, F oder ein aliphatischer und/oder aromatischer Kohlenwasserstoffrest mit 1 bis 20 C-Atomen, in dem auch H-Atome durch F ersetzt sein können; dabei können zwei oder mehrere benachbarte Substituenten $R^5$ auch miteinander ein mono- oder polycyclisches, aliphatisches Ringsystem bilden, das jedoch keine kondensierten aromatischen Ringe aufweist;

h ist 1;

i ist 0;

n ist 0 oder 1,

mit der Maßgabe, dass

die Gruppe $L^1$ und/oder $L^2$ mit dem Carbazolring der Formel (I) und der Dibenzofuranstruktur (Y=O) und/oder der Dibenzothiophenstruktur (Y=S) eine durchgängige Konjugation ausbildet.

**2.** Verbindung gemäß Anspruch 1, **dadurch gekennzeichnet, dass** n=0 ist.

**3.** Verbindungen gemäß mindestens einem der vorhergehenden Ansprüche, **dadurch gekennzeichnet, dass** mindestens eine der Gruppen $L^1$ oder $L^2$ Phenylen ist.

**4.** Verbindungen gemäß mindestens einem der vorhergehenden Ansprüche, **dadurch gekennzeichnet, dass** mindestens ein Y in der Formel (I) für O steht.

**5.** Verbindungen gemäß mindestens einem der vorhergehenden Ansprüche, **dadurch gekennzeichnet, dass** beide Y in derFormel (I) für O stehen.

**6.** Verbindungen gemäß mindestens einem der vorhergehenden Ansprüche, **dadurch gekennzeichnet, dass** mindestens ein Y in der Formel (I) für S steht.

**7.** Verbindungen gemäß mindestens einem der vorhergehenden Ansprüche, **dadurch gekennzeichnet, dass** mindestens ein Rest $R^1$ für eine Gruppe steht, die ausgewählt ist aus den Formeln ($R^1$-1) bis ($R^1$-22)

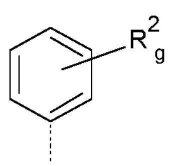

Formel ($R^1$-1)

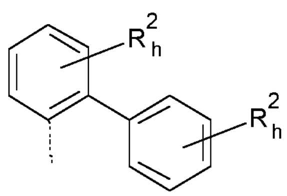

Formel ($R^1$-2)

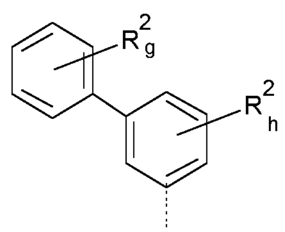

Formel ($R^1$-3)

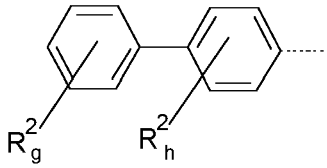

Formel ($R^1$-4)

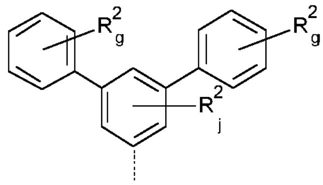

Formel ($R^1$-5)

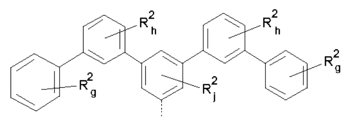

Formel ($R^1$-6)

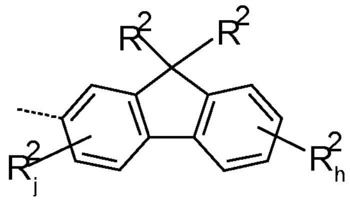

Formel ($R^1$-7)

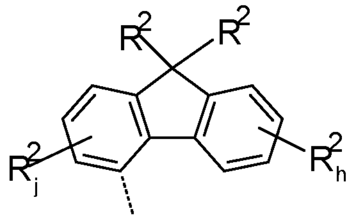

Formel ($R^1$-8)

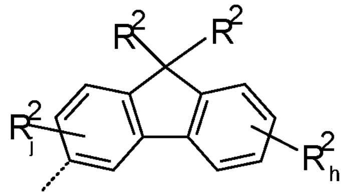

Formel ($R^1$-9)

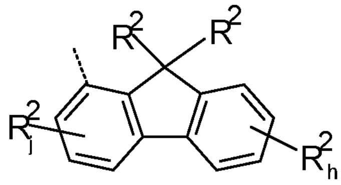

Formel ($R^1$-10)

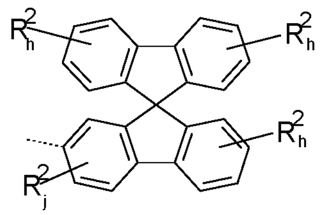

Formel ($R^1$-11)

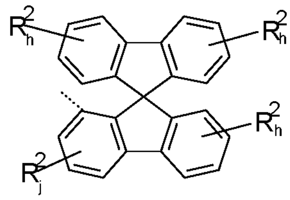

Formel ($R^1$-12)

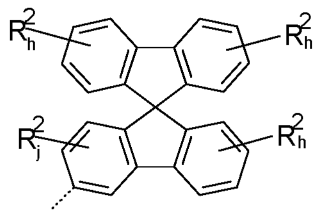

Formel ($R^1$-13)

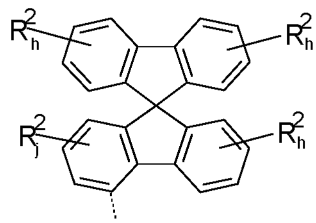

Formel ($R^1$-14)

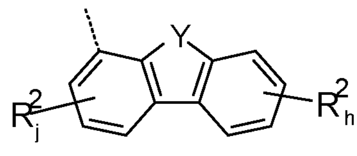

Formel ($R^1$-15)

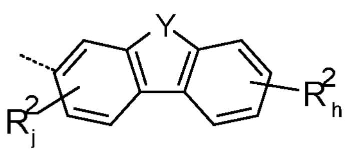

Formel ($R^1$-16)

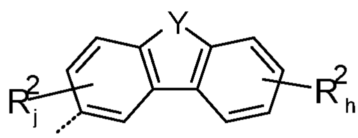

Formel ($R^1$-17)

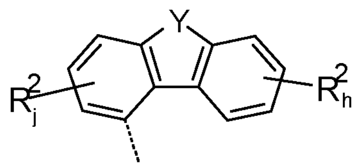

Formel ($R^1$-18)

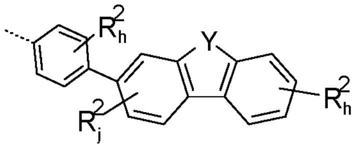

Formel ($R^1$-19)

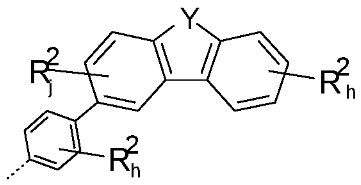

Formel ($R^1$-20)

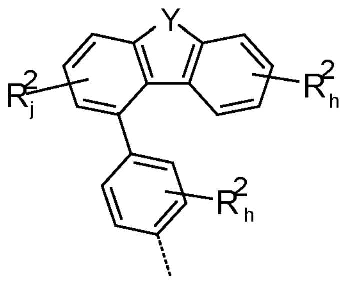

Formel ($R^1$-21)

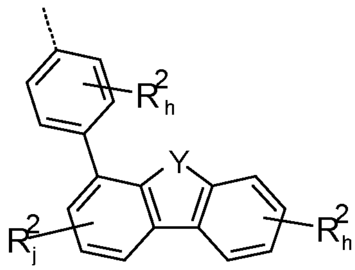

Formel ($R^1$-22)

, wobei die gestrichelte Bindung die Anbindungsposition markiert, Index g 0, 1, 2, 3, 4 oder 5 ist, der Index h 0, 1, 2, 3 oder 4 ist, der Index j 0, 1, 2 oder 3 ist, und $R^2$ und Y die in Anspruch 1 genannte Bedeutung aufweisen.

**8.** Verbindungen gemäß mindestens einem der vorhergehenden Ansprüche, **dadurch gekennzeichnet, dass** die Verbindung Strukturen der Formel (II)

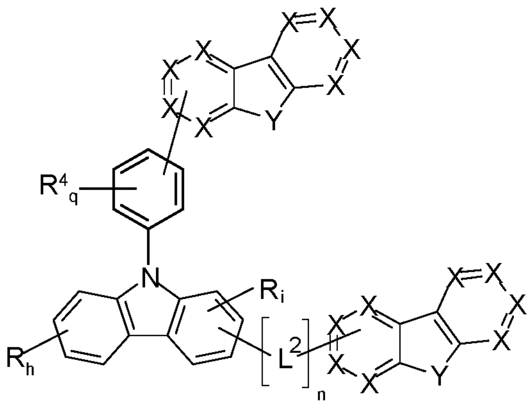

Formel (II)

umfasst, wobei die dargelegten Symbole X, Y, R, $R^4$, $L^1$, $L^2$ und die Indices h, i und n die in Anspruch 1 dargelegte Bedeutung haben, der Index q 0, 1, 2, 3 oder 4 bedeutet.

**9.** Verbindungen nach Anspruch 1 gemäß der folgenden Formeln

| | | |
|---|---|---|
| 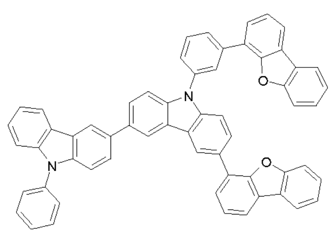 | 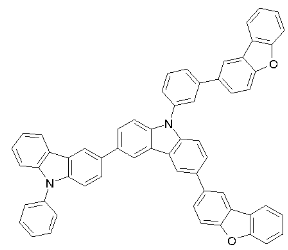 | 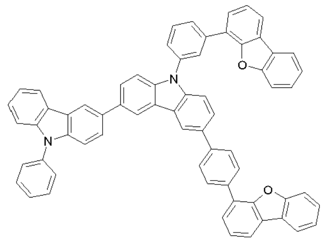 |
| Formel 25 | Formel 26 | Formel 27 |
| 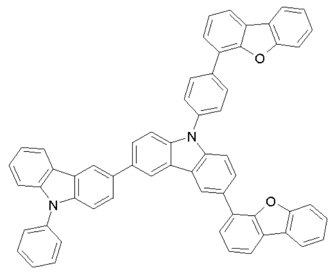 | 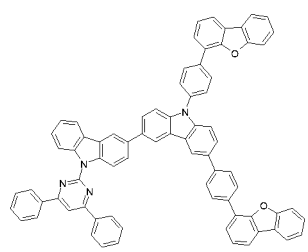 | 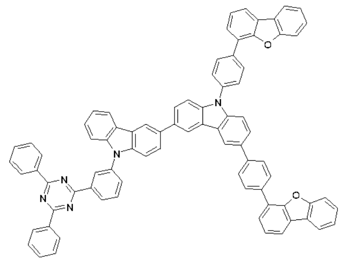 |
| Formel 28 | Formel 29 | Formel 30 |
| 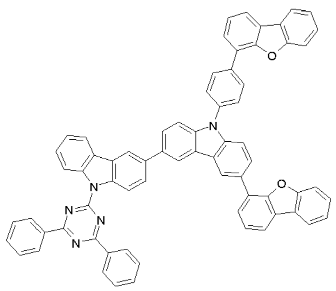 | 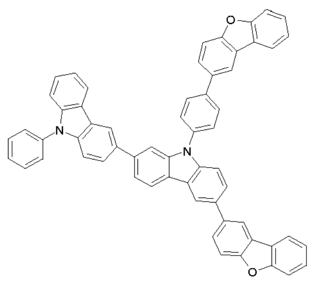 | 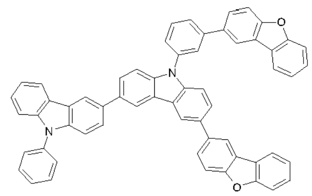 |
| Formel 37 | Formel 51 | Formel 75 |
| 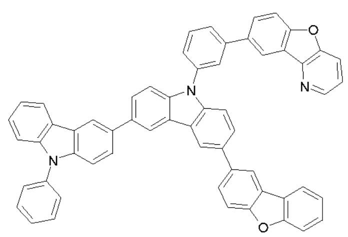 | 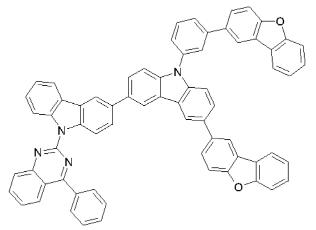 | 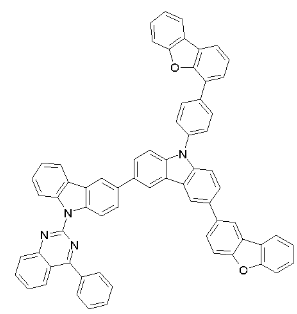 |
| Formel 79 | Formel 81 | Formel 82 |
| 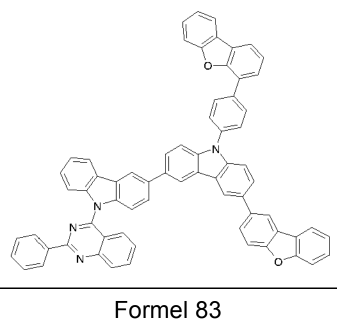 | 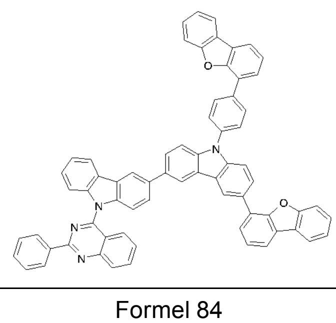 | |
| Formel 83 | Formel 84 | |

(fortgesetzt)

| | | |
|---|---|---|
| 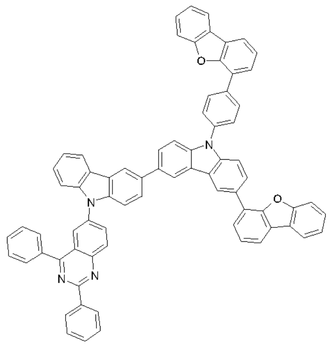 | 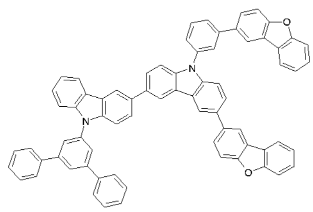 | 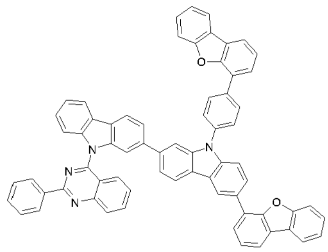 |
| Formel 85 | Formel 86 | Formel 87 |
| 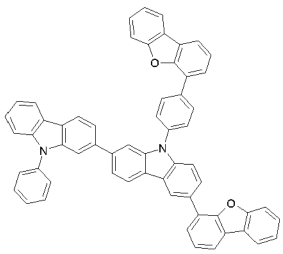 | 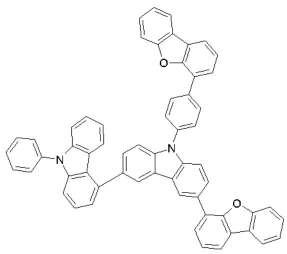 | 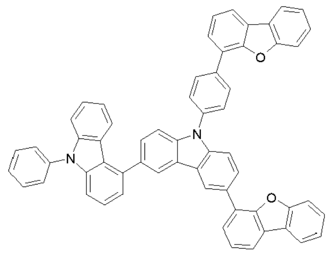 |
| Formel 88 | Formel 89 | Formel 140. |

**10.** Zusammensetzung enthaltend wenigstens eine Verbindung nach einem oder mehreren der Ansprüche 1 bis 9 und wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

**11.** Formulierung enthaltend mindestens eine Verbindung nach einem oder mehreren der Ansprüche 1 bis 9 und/oder wenigstens eine Zusammensetzung nach Anspruch 10 und mindestens ein Lösungsmittel.

**12.** Verwendung einer Verbindung nach einem oder mehreren der Ansprüche 1 bis 9 oder einer Zusammensetzung nach Anspruch 10 in einer elektronischen Vorrichtung als Lochblockiermaterial, Elektroneninjektionsmaterial, und/oder Elektronentransportmaterial.

**13.** Elektronische Vorrichtung, enthaltend mindestens eine Verbindung nach einem oder mehreren der Ansprüche 1 bis 9 oder eine Zusammensetzung gemäß Anspruch 10, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laser-dioden.

**14.** Verwendung einer Verbindung nach einem oder mehreren der Ansprüche 1 bis 9 als Komponente von Mixed-Matrix-Systemen, die zwei oder drei verschiedene Matrixmaterialien umfassen.

## Claims

**1.** Compound containing structures of the formula (I),

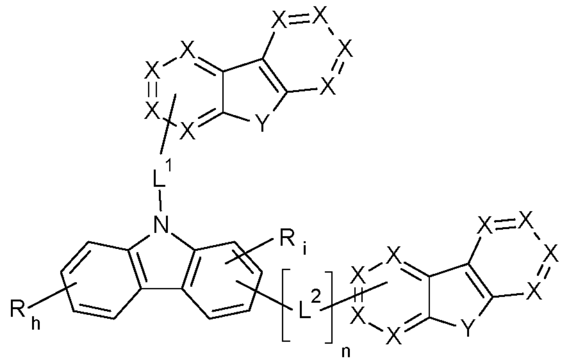

formula (I)

where the following applies to the symbols used:

X is $CR^1$ or C for the bonding site of the radicals $L^1$, $L^2$ or of the carbazole group;
Y is on each occurrence, identically or differently, O or S;
$L^1$, $L^2$ is an aromatic ring system having 6 to 40 C atoms which contains no condensed aromatic rings, where the aromatic ring system may be substituted by one or more radicals $R^4$, where the substituents $R^4$ likewise contain no condensed aromatic rings and likewise contain no heteroaromatic ring systems or heteroaryl groups;

one of the radicals R is a carbazole group which is not bonded via the nitrogen of the carbazole group and which may in each case be substituted by one or more radicals $R^2$, and all other radicals R are equal to H;

$R^1$ is on each occurrence, identically or differently, H, D, F, Cl, Br, I, CHO, $C(=O)A_r{}^1$, $P(=O)(A_r{}^1)_2$, $S(=O)A_r{}^1$, $S(=O)_2Ar^1$, CN, $NO_2$, $Si(R^2)_3$, $B(OR^2)_2$, $OSO_2R^2$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^2$, where one or more non-adjacent $CH_2$ groups may be replaced by $R^2C=CR^2$, C=C , $Si(R^2)_2$, $Ge(R^2)_2$, $Sn(R^2)_2$, C=O, C=S, C=Se, $P(=O)(R^2)$, SO, $SO_2$, O, S or $CONR^2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or a combination of these systems; two or more adjacent substituents $R^1$ may also form a mono- or polycyclic, aliphatic or aromatic ring system with one another;
$R^2$ is on each occurrence, identically or differently, H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by C=C , $Si(R^3)_2$, $Ge(R^3)_2$, $Sn(R^3)_2$, C=O, C=S, C=Se, $P(=O)(R^3)$, SO, $SO_2$, O, S or $CONR^3$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or a combination of these systems; two or more adjacent substituents $R^2$ may also form a mono- or polycyclic, aliphatic or aromatic ring system with one another;
$Ar^1$ is on each occurrence, identically or differently, an aromatic or heteroaromatic ring system having 5 to 30 aromatic ring atoms, which may be substituted by one or more radicals $R^2$; two radicals $Ar^1$ which are bonded to the same phosphorus atom may also be linked to one another by a single bond or a bridge selected from $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ and $P(=O)R^3$;
$R^3$ is on each occurrence, identically or differently, H, D, F or an aliphatic, aromatic and/or heteroaromatic hydrocarbon radical having 1 to 20 C atoms, in which, in addition, H atoms may be replaced by F; two or more adjacent substituents $R^3$ may also form a mono- or polycyclic, aliphatic or aromatic ring system with one another;
$R^4$ is on each occurrence, identically or differently, H, D, F, Cl, Br, I, CHO, $C(=O)Ar^2$, $P(=O)(Ar^2)_2$, $S(=O)Ar^2$, $S(=O)_2Ar^2$, CN, $NO_2$, $Si(R^5)_3$, $B(OR^5)_2$, $OSO_2R^5$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^5$, where one or more non-adjacent $CH_2$ groups may be replaced by C=C , $Si(R^5)_2$, $Ge(R^5)_2$, $Sn(R^5)_2$, C=O, C=S, C=Se, $P(=O)(R^5)$, SO, $SO_2$, O, S or $CONR^5$ and where one or more H atoms may be replaced by D, F, Cl, Br, I, CN or $NO_2$, or an aromatic ring system having 5 to 40 C atoms

which contains no condensed aromatic rings and which may in each case be substituted by one or more radicals $R^5$, or an aryloxy group having 5 to 40 aromatic ring atoms, which may be substituted by one or more radicals $R^5$, or a combination of these systems; two or more adjacent substituents $R^4$ may also form a mono- or polycyclic, aliphatic ring system with one another, which, however, contains no condensed aromatic rings;
$Ar^2$ is on each occurrence, identically or differently, an aromatic ring system having 5 to 30 C atoms, which contains no condensed aromatic rings and which may be substituted by one or more radicals $R^5$;
$R^5$ is on each occurrence, identically or differently, H, D, F or an aliphatic and/or aromatic hydrocarbon radical having 1 to 20 C atoms, in which, in addition, H atoms may be replaced by F; two or more adjacent substituents $R^5$ may also form a mono- or polycyclic, aliphatic ring system with one another, which, however, contains no condensed aromatic rings;
h is 1;
i is 0;
n is 0 or 1;

with the proviso that
the group $L^1$ and/or $L^2$ forms a continuous conjugation with the carbazole ring of the formula (I) and the dibenzofuran structure (Y=O) and/or the dibenzothiophene structure (Y=S).

2. Compound according to Claim 1, **characterised in that** n=0.

3. Compounds according to at least one of the preceding claims, **characterised in that** at least one of the groups $L^1$ or $L^2$ is phenylene.

4. Compounds according to at least one of the preceding claims, **characterised in that** at least one Y in the formula (I) stands for O.

5. Compounds according to at least one of the preceding claims, **characterised in that** both Y in the formula (I) stand for O.

6. Compounds according to at least one of the preceding claims, **characterised in that** at least one Y in the formula (I) stands for S.

7. Compounds according to at least one of the preceding claims, **characterised in that** at least one radical $R^1$ stands for a group selected from the formulae ($R^1$-1) to ($R^1$-22)

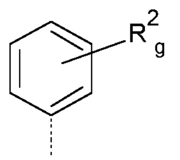

formula ($R^1$-1)

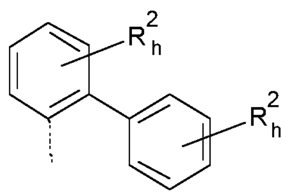

formula ($R^1$-2)

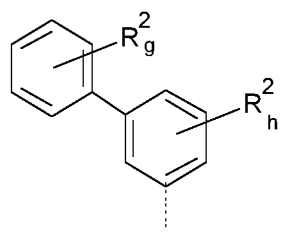

formula ($R^1$-3)

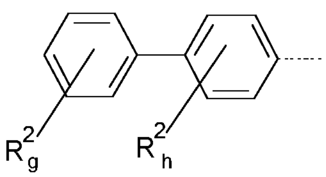

formula ($R^1$-4)

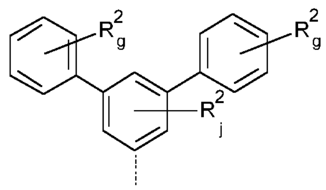

formula ($R^1$-5)

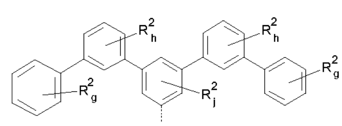

formula ($R^1$-6)

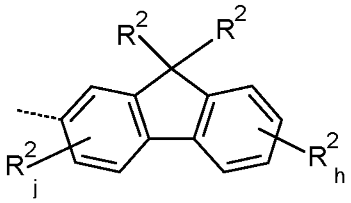

formula ($R^1$-7)

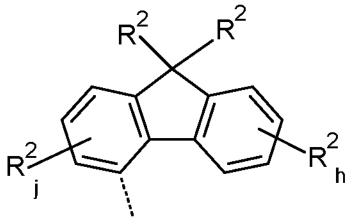

formula ($R^1$-8)

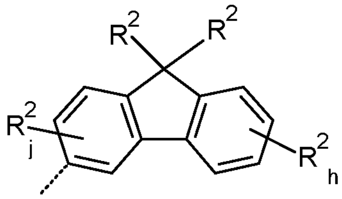

formula ($R^1$-9)

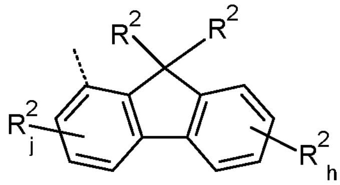

formula ($R^1$-10)

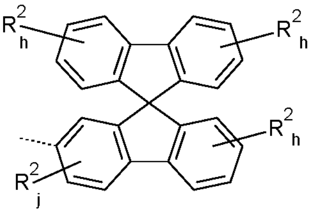

formula ($R^1$-11)

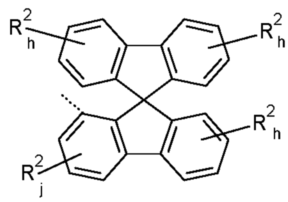

formula ($R^1$-12)

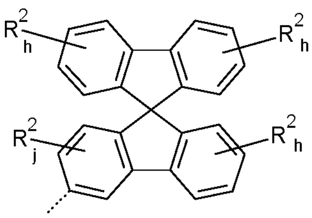

formula ($R^1$-13)

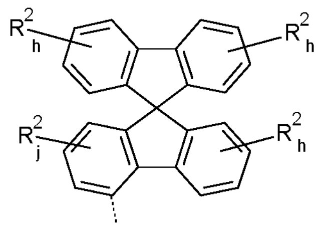

formula ($R^1$-14)

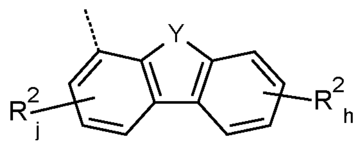

formula ($R^1$-15)

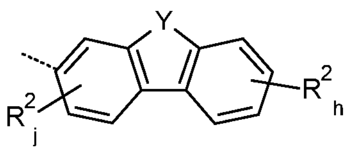

formula ($R^1$-16)

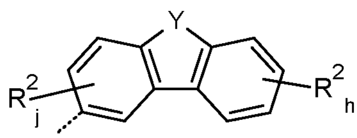

formula ($R^1$-17)

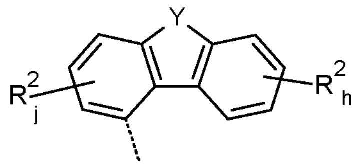

formula ($R^1$-18)

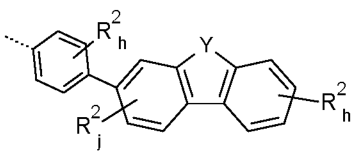

formula ($R^1$-19)

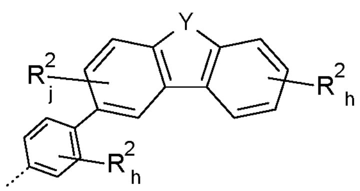

formula ($R^1$-20)

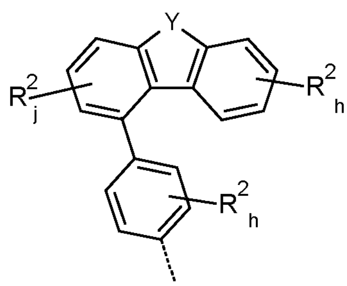

formula ($R^1$-21)

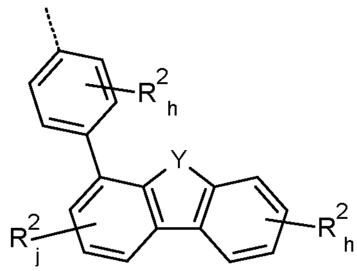

formula ($R^1$-22)

where the dashed bond marks the bonding position, index g is 0, 1, 2, 3, 4 or 5, the index h is 0, 1, 2, 3 or 4, the index j is 0, 1, 2 or 3, and $R^2$ and Y have the meaning given in Claim 1.

**8.** Compounds according to at least one of the preceding claims, **characterised in that** the compound contains structures of the formula (II)

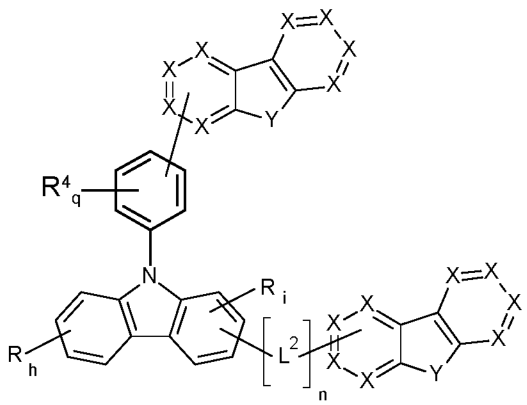

formula (II)

where the symbols X, Y, R, $R^4$, $L^1$, $L^2$ shown and the indices h, i and n have the meaning given in Claim 1, the index q denotes 0, 1, 2, 3 or 4.

**9.** Compounds according to Claim 1 of the following formulae

| | | |
|---|---|---|
| 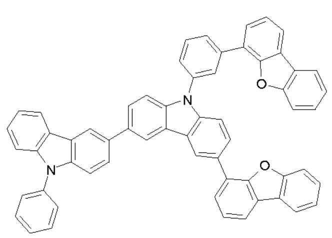 | 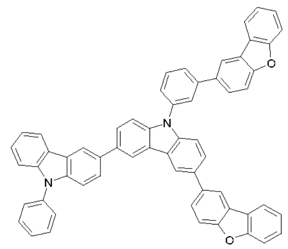 | 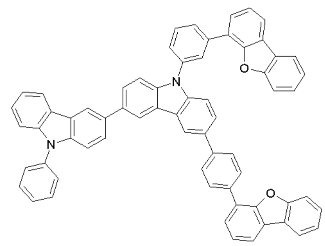 |
| formula 25 | formula 26 | formula 27 |

(continued)

| | | |
|---|---|---|
| 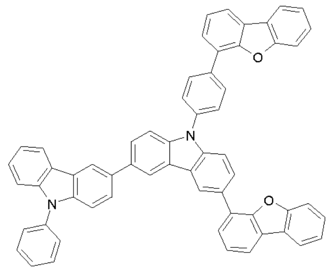 | 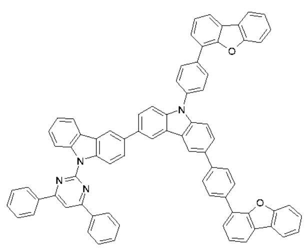 | 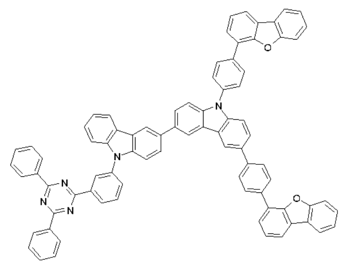 |
| formula 28 | formula 29 | formula 30 |
| 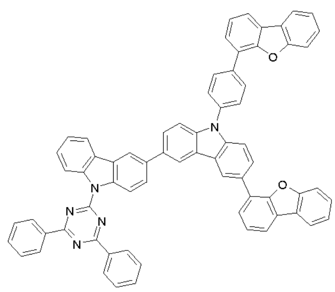 | 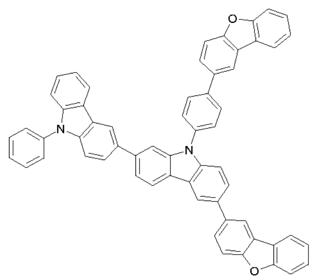 | 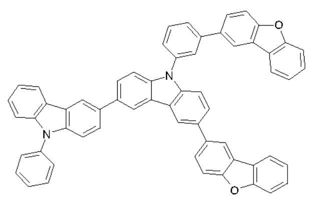 |
| formula 37 | formula 51 | formula 75 |
| 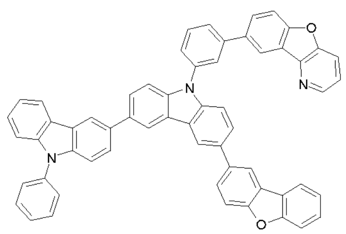 | 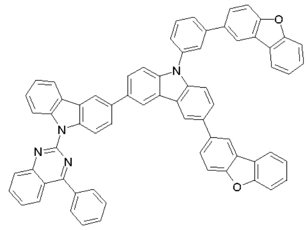 | 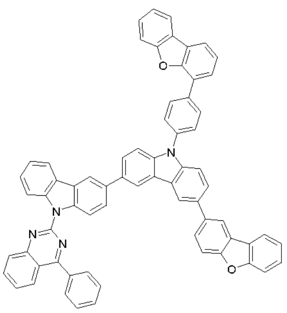 |
| formula 79 | formula 81 | formula 82 |
| 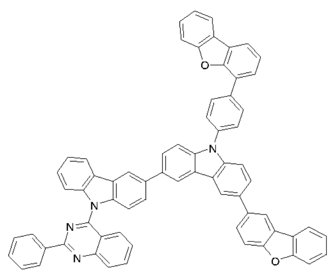 | 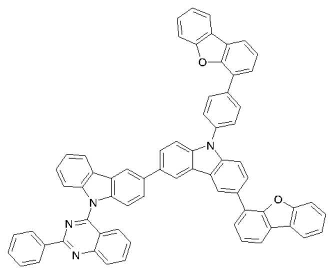 | |
| formula 83 | formula 84 | |

(continued)

| | | |
|---|---|---|
| 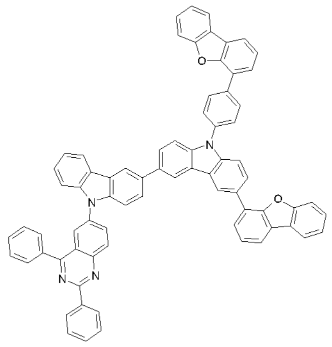 | 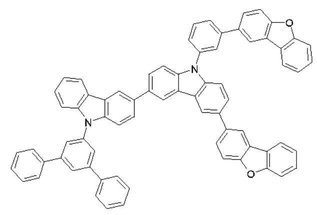 | 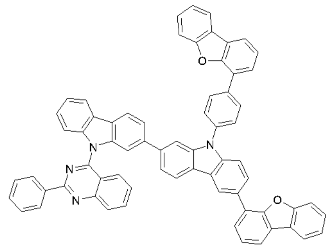 |
| formula 85 | formula 86 | formula 87 |
| 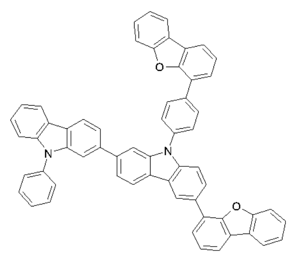 | 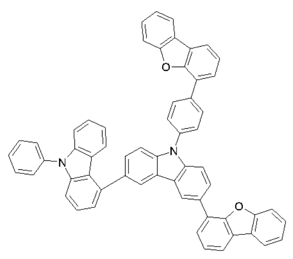 | 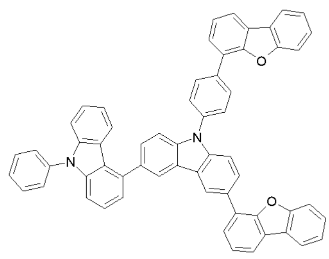 |
| formula 88 | formula 89 | formula 140. |

**10.** Composition comprising at least one compound according to one or more of Claims 1 to 9 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**11.** Formulation comprising at least one compound according to one or more of Claims 1 to 9 and/or at least one composition according to Claim 10 and at least one solvent.

**12.** Use of a compound according to one or more of Claims 1 to 9 or a composition according to Claim 10 in an electronic device as hole-blocking material, electron-injection material and/or electron-transport material.

**13.** Electronic device comprising at least one compound according to one or more of Claims 1 to 9 or a composition according to Claim 10, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells and organic laser diodes.

**14.** Use of a compound according to one or more of Claims 1 to 9 as component of mixed-matrix systems which comprise two or three different matrix materials.

**Revendications**

**1.** Composé contenant des structures de formule (I),

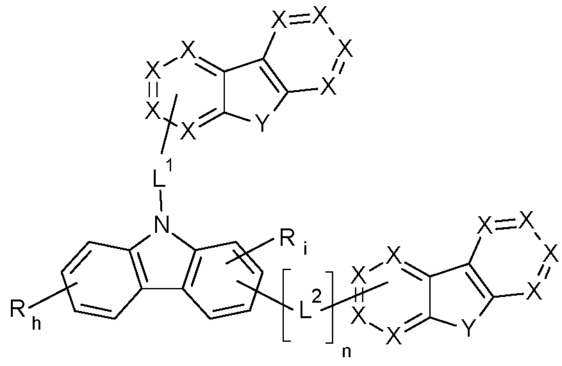

formule (I)

dans lequel ce qui suit s'applique aux symboles utilisés :

X est $CR^1$ ou C pour le site de fixation des radicaux $L^1$, $L^2$ ou du groupement carbazole ;
Y est à chaque occurrence, de manière identique ou différente, O ou S ;
$L^1$, $L^2$ est un noyau aromatique ayant de 6 à 40 atomes de C qui ne contient aucun cycle aromatique condensé, où le noyau aromatique peut être substitué par un ou plusieurs radicaux $R^4$, où les substituants $R^4$ ne contiennent de même aucun cycle aromatique condensé et ne contiennent de même aucun noyau hétéroaromatique ou groupements hétéroaryle ;

l'un des radicaux R est un groupement carbazole qui n'est pas fixé via l'azote du groupement carbazole et qui peut dans chaque cas être substitué par un ou plusieurs radicaux $R^2$, et tous les autres radicaux R sont égaux à H ;

$R^1$ est à chaque occurrence, de manière identique ou différente, H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^2)_3$, $B(OR^2)_2$, $OSO_2R^2$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut être dans chaque cas substitué par un ou plusieurs radicaux $R^2$, où un ou plusieurs groupements $CH_2$ non adjacents peuvent être remplacés par $R^2C=CR^2$, C≡C, $Si(R^2)_2$, $Ge(R^2)_2$, $Sn(R^2)_2$, C=O, C=S, C=Se, $P(=O)(R^2)$, SO, $SO_2$, O, S ou $CONR^2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut être dans chaque cas substitué par un ou plusieurs radicaux $R^2$, ou un groupement aryloxy ou hétéroaryloxy ayant de 5 à 40 atomes de cycle aromatique, qui peut être substitué par un ou plusieurs radicaux $R^2$, ou une combinaison de ces systèmes ; deux, ou plus, substituants $R^1$ adjacents peuvent également former un noyau mono- ou polycyclique, aliphatique ou aromatique, l'un avec l'autre ;
$R^2$ est à chaque occurrence, de manière identique ou différente, H, D, F, Cl, Br, I, CHO, $C(=O)A_r{}^1$, $P(=O)(Ar^1)_2$, $S(=O)A_r{}^1$, $S(=O)_2Ar^1$, CN, $NO_2$, $Si(R^3)_3$, $B(OR^3)_2$, $OSO_2R^3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut être dans chaque cas substitué par un ou plusieurs radicaux $R^3$, où un ou plusieurs groupements $CH_2$ non adjacents peuvent être remplacés par C=C , $Si(R^3)_2$, $Ge(R^3)_2$, $Sn(R^3)_2$, C=O, C=S, C=Se, $P(=O)(R^3)$, SO, $SO_2$, O, S ou $CONR^3$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut être dans chaque cas substitué par un ou plusieurs radicaux $R^3$, ou un groupement aryloxy ou hétéroaryloxy ayant de 5 à 40 atomes de cycle aromatique, qui peut être substitué par un ou plusieurs radicaux $R^3$, ou une combinaison de ces systèmes ; deux, ou plus, substituants $R^2$ adjacents peuvent également former un noyau mono- ou polycyclique, aliphatique ou aromatique, l'un avec l'autre ;
$Ar^1$ est à chaque occurrence, de manière identique ou différente, un noyau aromatique ou hétéroaromatique ayant de 5 à 30 atomes de cycle aromatique, qui peut être substitué par un ou plusieurs radicaux $R^2$ ; deux radicaux $Ar^1$ qui sont fixés au même atome de phosphore peuvent également être fixés l'un à l'autre par une liaison simple ou un pont choisi parmi $B(R^3)$, $C(R^3)_2$, $Si(R^3)_2$, C=O, $C=NR^3$, $C=C(R^3)_2$, O, S, S=O, $SO_2$, $N(R^3)$, $P(R^3)$ et $P(=O)R^3$ ;
$R^3$ est à chaque occurrence, de manière identique ou différente, H, D, F ou un radical hydrocarboné aliphatique, aromatique et/ou hétéroaromatique ayant de 1 à 20 atomes de C, dans lequel, en outre, des atomes de H peuvent être remplacés par F ; deux, ou plus, substituants $R^3$ adjacents peuvent également former un noyau mono- ou polycyclique, aliphatique ou aromatique, l'un avec l'autre ;
$R^4$ est à chaque occurrence, de manière identique ou différente, H, D, F, Cl, Br, I, CHO, $C(=O)Ar^2$, $P(=O)(Ar^2)_2$,

$S(=O)Ar^2$, $S(=O)_2Ar^2$, CN, $NO_2$, $Si(R^5)_3$, $B(OR^5)_2$, $OSO_2R^5$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut être dans chaque cas substitué par un ou plusieurs radicaux $R^5$, où un ou plusieurs groupements $CH_2$ non adjacents peuvent être remplacés par C=C , $Si(R^5)_2$, $Ge(R^5)_2$, $Sn(R^5)_2$, C=O, C=S, C=Se, $P(=O)(R^5)$, SO, $SO_2$, O, S ou $CONR^5$ et où un ou plusieurs atomes de H peuvent être remplacés par D, F, Cl, Br, I, CN ou $NO_2$, ou un noyau aromatique ayant de 5 à 40 atomes de C qui ne contient aucun cycle aromatique condensé et qui peut être dans chaque cas substitué par un ou plusieurs radicaux $R^5$, ou un groupement aryloxy ayant de 5 à 40 atomes de cycle aromatique, qui peut être substitué par un ou plusieurs radicaux $R^5$, ou une combinaison de ces systèmes ; deux, ou plus, substituants $R^4$ adjacents peuvent également former un noyau monoou polycyclique aliphatique l'un avec l'autre, qui ne contient cependant aucun cycle aromatique condensé ;
$Ar^2$ est à chaque occurrence, de manière identique ou différente, un noyau aromatique ayant de 5 à 30 atomes de C, qui ne contient aucun cycle aromatique condensé et qui peut être substitué par un ou plusieurs radicaux $R^5$ ;
$R^5$ est à chaque occurrence, de manière identique ou différente, H, D, F ou un radical hydrocarboné aliphatique et/ou aromatique ayant de 1 à 20 atomes de C, dans lequel, en outre, des atomes de H peuvent être remplacés par F ; deux, ou plus, substituants $R^5$ adjacents peuvent également former un noyau mono- ou polycyclique aliphatique l'un avec l'autre, qui ne contient cependant aucun cycle aromatique condensé ;
h vaut 1 ;
i vaut 0 ;
n vaut 0 ou 1 ;

à condition que
le groupement $L^1$ et/ou $L^2$ forme une conjugaison continue avec le cycle carbazole de formule (I) et la structure de dibenzofuranne (Y=O) et/ou la structure de dibenzothiophène (Y=S).

2. Composé selon la revendication 1, **caractérisés en ce que** n=0.

3. Composés selon au moins l'une des revendications précédentes, **caractérisés en ce qu'**au moins l'un des groupements $L^1$ ou $L^2$ est phénylène.

4. Composés selon au moins l'une des revendications précédentes, **caractérisés en ce qu'**au moins un Y dans la formule (I) représente O.

5. Composés selon au moins l'une des revendications précédentes, **caractérisés en ce que** les deux Y dans la formule (I) représentent O.

6. Composés selon au moins l'une des revendications précédentes, **caractérisés en ce qu'**au moins un Y dans la formule (I) représente S.

7. Composés selon au moins l'une des revendications précédentes, **caractérisés en ce qu'**au moins un radical $R^1$ représente un groupement choisi parmi les formules ($R^1$-1) à ($R^1$-22)

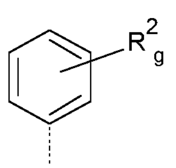
formule ($R^1$-1)

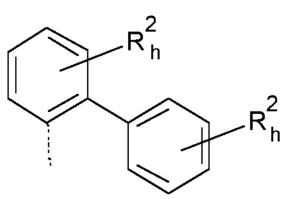
formule ($R^1$-2)

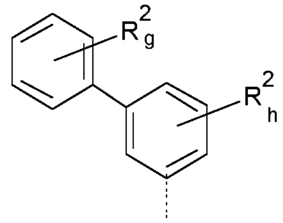
formule ($R^1$-3)

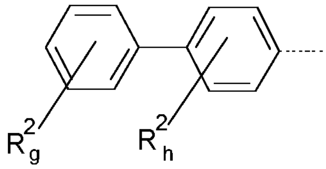

formule ($R^1$-4)

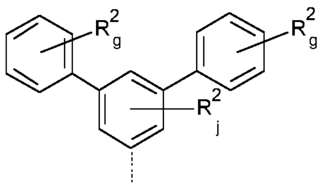

formule ($R^1$-5)

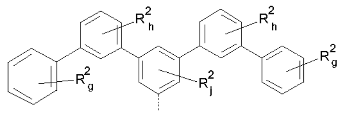

formule ($R^1$-6)

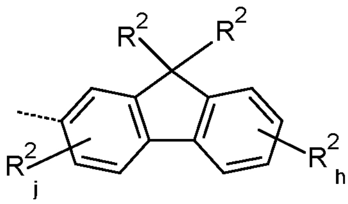

formule ($R^1$-7)

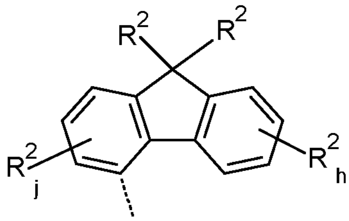

formule ($R^1$-8)

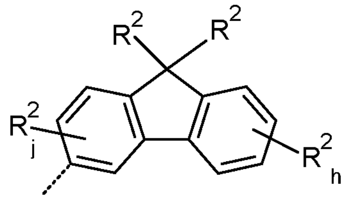

formule ($R^1$-9)

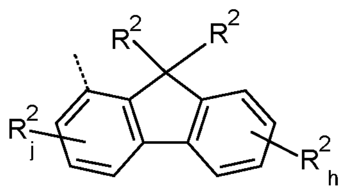

formule ($R^1$-10)

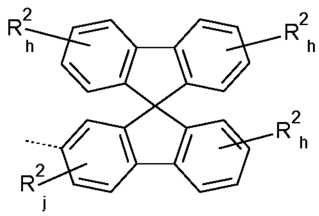

formule ($R^1$-11)

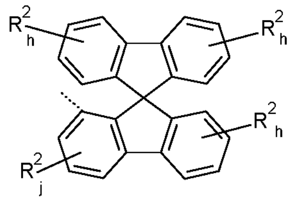

formule ($R^1$-12)

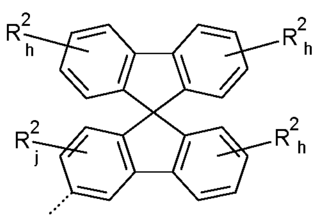

formule ($R^1$-13)

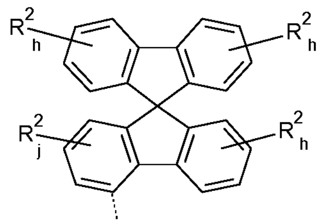

formule ($R^1$-14)

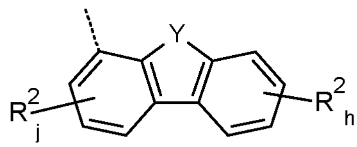

formule ($R^1$-15)

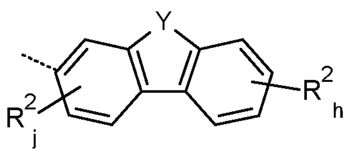

formule ($R^1$-16)

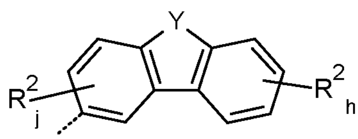

formule ($R^1$-17)

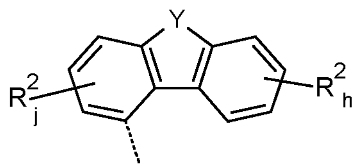

formule ($R^1$-18)

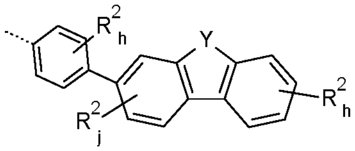

formule ($R^1$-19)

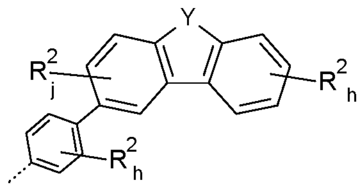

formule ($R^1$-20)

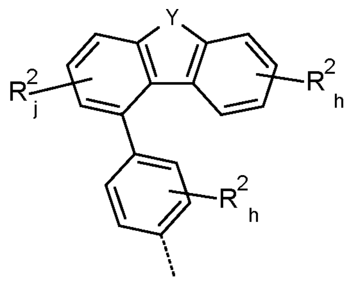

formule ($R^1$-21)

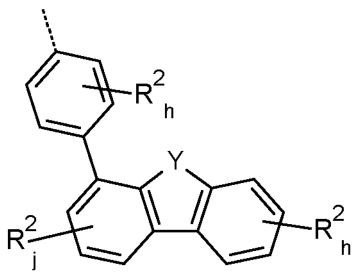

formule ($R^1$-22)

dans lesquelles la liaison en pointillés marque la position de liaison, l'indice g vaut 0, 1, 2, 3, 4 ou 5, l'indice h vaut 0, 1, 2, 3 ou 4, l'indice j vaut 0, 1, 2 ou 3, et $R^2$ et Y revêtent la signification donnée selon la revendication 1.

**8.** Composés selon au moins l'une des revendications précédentes, **caractérisés en ce que** le composé contient des structures de formule (II)

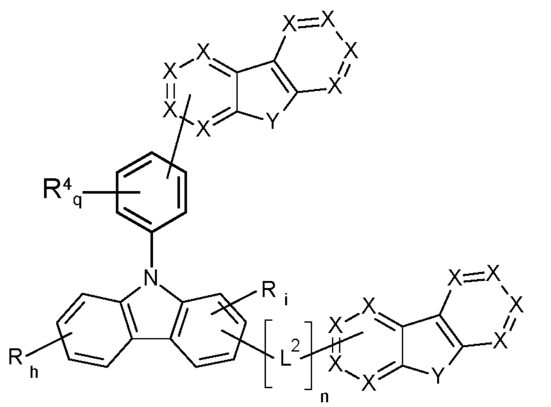

formule (II)

dans laquelle les symboles X, Y, R, $R^4$, $L^1$, $L^2$ illustrés et les indices h, i et n revêtent la signification donnée selon la revendication 1, l'indice q désigne 0, 1, 2, 3 ou 4.

**9.** Composés selon la revendication 1, répondant aux formules suivantes

| | | |
|---|---|---|
| 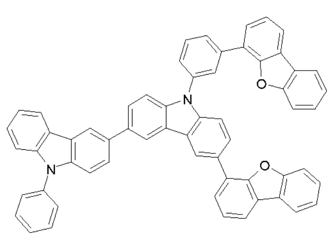 | 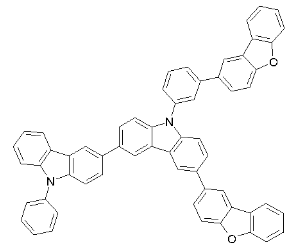 | 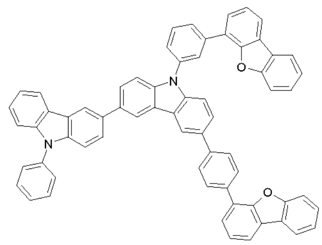 |
| formule 25 | formule 26 | formule 27 |
| 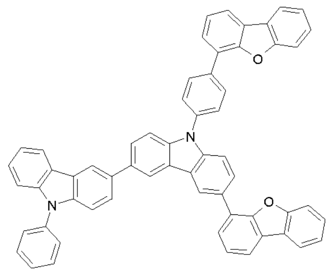 | 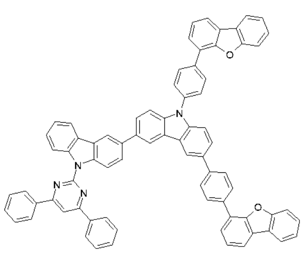 | 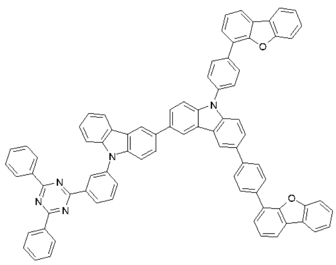 |
| formule 28 | formule 29 | formule 30 |
| 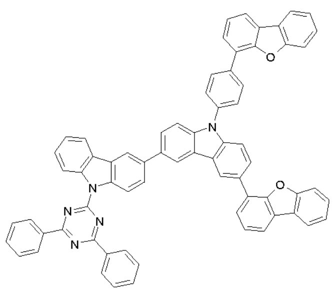 | 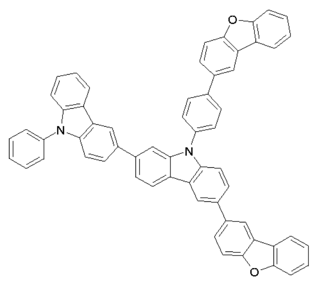 | 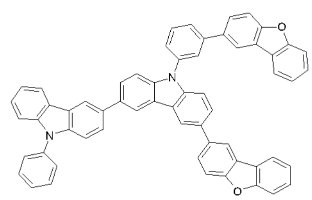 |
| formule 37 | formule 51 | formule 75 |
| 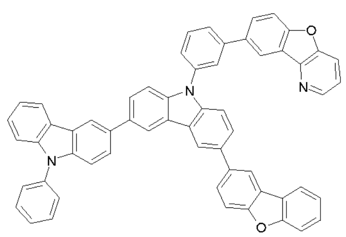 | 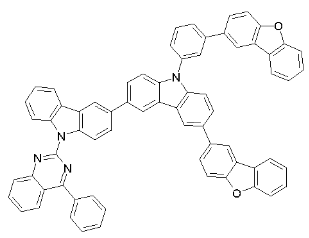 | 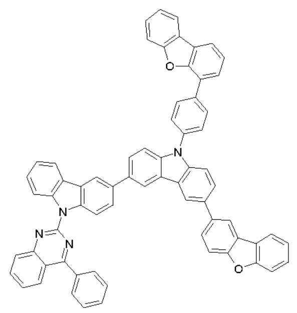 |
| formule 79 | formule 81 | formule 82 |
| 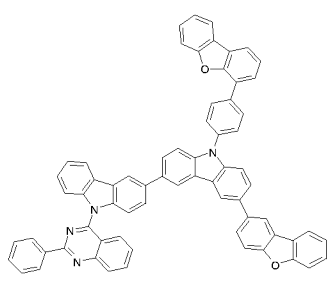 | 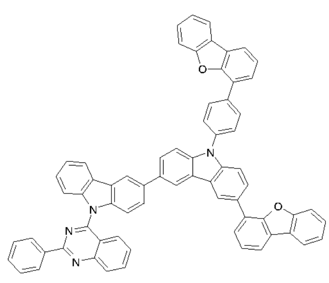 | |
| formule 83 | formule 84 | |

(suite)

| | | |
|---|---|---|
| 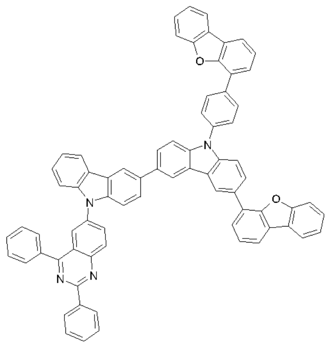 | 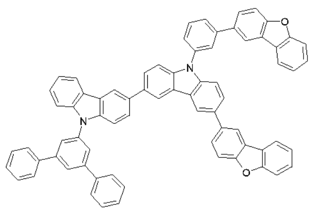 | 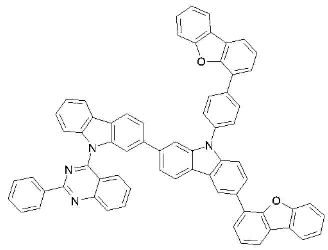 |
| formule 85 | formule 86 | formule 87 |
| 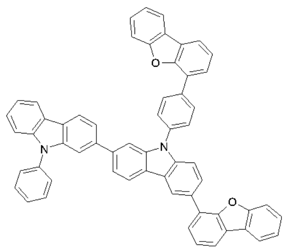 | 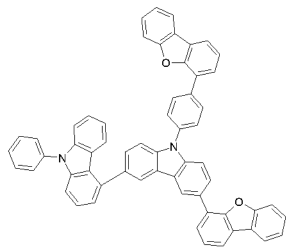 | 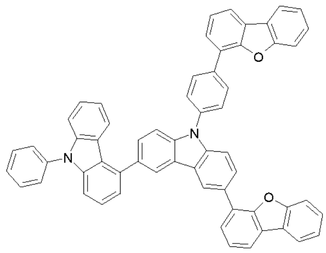 |
| formule 88 | formule 89 | formule 140. |

**10.** Composition comprenant au moins un composé selon l'une ou plusieurs parmi les revendications 1 à 9 et au moins un autre composé choisi dans le groupe constitué par les émetteurs fluorescents, les émetteurs phosphorescents, les matériaux hôtes, les matériaux matriciels, les matériaux de transport d'électrons, les matériaux d'injection d'électrons, les matériaux conducteurs de trous, les matériaux d'injection de trous, les matériaux de blocage d'électrons et les matériaux de blocage de trous.

**11.** Formulation comprenant au moins un composé selon l'une ou plusieurs parmi les revendications 1 à 9 et/ou au moins une composition selon la revendication 10 et au moins un solvant.

**12.** Utilisation d'un composé selon l'une ou plusieurs parmi les revendications 1 à 9 ou d'une composition selon la revendication 10, dans un dispositif électronique comme matériau de blocage de trous, matériau d'injection d'électrons et/ou matériau de transport d'électrons.

**13.** Dispositif électronique comprenant au moins un composé selon l'une ou plusieurs parmi les revendications 1 à 9 ou une composition selon la revendication 10, le dispositif électronique étant choisi de préférence dans le groupe constitué par les dispositifs électroluminescents organiques, les circuits intégrés organiques, les transistors organiques à effet de champ, les transistors organiques en couches minces, les transistors organiques émetteurs de lumière, les cellules solaires organiques, les détecteurs optiques organiques, les photorécepteurs organiques, les dispositifs organiques à extinction de champ, les cellules électrochimiques émettrices de lumière et les diodes laser organiques.

**14.** Utilisation d'un composé selon l'une ou plusieurs parmi les revendications 1 à 9, comme composant de systèmes à matrice mixte qui comprennent deux ou trois matériaux matriciels différents.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9013148 A1
- WO 2008146838 A
- JP 2011008991 B
- WO 2009060757 A
- WO 2011004639 A
- US 20120289708 A
- WO 2012086170 A
- US 2012071668 A
- US 2012091887 A
- WO 2012033108 A
- CN 102850334
- WO 2013032278 A
- US 20120256169 A
- WO 2012036482 A
- WO 20135923 A
- WO 2013084885 A
- WO 2013102992 A
- WO 2013084881 A
- WO 2012067425 A
- US 2011260138 A
- WO 2011125680 A
- WO 2011122132 A
- WO 2013109045 A
- WO 2013151297 A
- WO 201341176 A
- WO 2012108389 A
- KR 20130025087
- KR 20130112342
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2005011013 A
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- US 20090134784 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 2005111172 A
- WO 2006117052 A
- WO 2010054729 A
- WO 2010054730 A
- WO 2010015306 A
- EP 652273 A
- WO 2009062578 A
- WO 2009148015 A
- US 20090136779 A
- WO 2010050778 A
- WO 2011042107 A
- WO 2011088877 A
- WO 2010108579 A
- WO 2004058911 A


### In der Beschreibung aufgeführte Nicht-Patentliteratur


- **M. S. ARNOLD et al.** *Appl. Phys. Lett.*, 2008, vol. 92, 053301